# EUROPEAN PATENT APPLICATION

(11) **EP 3 466 539 A1**
(43) Date of publication of application: **10.04.2019**
(21) Application number: 17840471.1
(22) Date of filing: 13.10.2017
(51) Int. Cl.: B01J 31/12, B01J 37/34, B01J 35/08, C07C 51/12, C07C 53/08

(54) **HETEROGENEOUS CATALYST FOR PRODUCING ACETIC ACID BY METHANOL CARBONYLATION**

(30) Priority: 23.08.2017 KR 20170106592
(71) Applicant: Poohung Photo-Chemical Co., Ltd., Gyeonggi-do 15434 (KR)
(72) Inventor: KOH, Jae Cheon, Anyang-si Gyeonggi-do 13943 (KR); WOO, Dong Wook, Yuseong-gu Darjeon 34082 (KR); PARK, Chang Geun, Yuseong-gu Daejeon 34116 (KR); BANG, Woong, Suwon-si Gyeonggi-do 16420 (KR)
(74) Representative: Izquierdo Blanco, Maria Alicia
(86) International application number: PCT/KR2017/011275
(87) International publication number: WO 2019/039648

(57) **Abstract**

Disclosed is a heterogeneous catalyst for producing acetic acid by carbonylation of methanol. In the heterogeneous catalyst, a rhodium complex ion is ionically bonded to an insoluble catalyst support, and the insoluble catalyst support includes a fluoropolymer having a quaternary pyridine radical alone or in combination with an acetate radical grafted on the surface thereof. According to the disclosure, a fixed-bed bubble column reactor can be easily designed. In addition, a special device for catalyst separation is not required, and thus the device manufacturing cost can be saved, the operating cost can be reduced due to process simplification, and productivity can be greatly increased.

## Description

### BACKGROUND

### 1. Technical Field

The present invention relates to a catalyst for producing acetic acid by carbonylation of methanol, and more particularly to a rhodium-based catalyst which is a heterogeneous catalyst for producing acetic acid by carbonylation of methanol.

### 2. Description of the Related Art

Among organic carbonate compounds, acetic acid is commonly known as vinegar acid. In the past, acetic acid was produced by fermentation of edible alcohol, whereas, currently, it is industrially produced in large amounts by various chemical processes. Acetic acid is used for the production of acetic anhydride which is used as a raw material for the production of either a vinyl acetate monomer which is a monomer for the production of polymers or cellulose acetate. In addition, acetic acid is also used as a raw material for the production of ethyl or butyl acetate which is used as a solvent. With industrial development, the demand for acetic acid is increasing. Since commercial plants for producing acetic acid from petrochemical raw materials in the presence of rhodium (Rh), iridium (Ir) and ruthenium (Ru) catalysts were constructed, this acetic acid production process has been repeatedly improved into processes having improved efficiency due to the development of various advanced catalyst technologies, and construction of acetic acid production plants has been accelerated. As a result, the total amount of acetic acid produced by the carbonylation reaction of methanol with carbon monoxide reached 6 million tons in 2015, accounting for 62% of total acetic acid production.

Accordingly, a process that is most widely used among acetic acid production processes is a process of adding carbon monoxide to methanol by the carbonylation reaction of methanol.

Methanol used as a raw material is produced from syngas in an easy and inexpensive way, and carbon monoxide used is isolated and purified from syngas.

Carbonylation of methanol involves the following reactions:

### Main reaction

CH₃OH + CO → CH₃COOH ΔH₂₉₈= -33kcal/gmol methanol ....... (1)

### Side reactions

CO + H₂O → CO₂ + H₂ (water-gas shift reaction) ......... (2)

CH₃OH + H₂ → CH₄ + H₂O (methanation reaction) ........... (3)

CO + 3H₂ → CH₄ + H₂O ................................... (4)

2CH₃OH → CH₃OCH₃ + H₂O ................................. (5)

CH₃OH + CH₃COOH ↔ CH₃COOCH₃ + H₂O ....................... (6)

CH₃COOH + 2H₂ → CH₃CH₂OH + H₂O .......................... (7)

CH₃CH₂OH + CO → CH₃CH₂COOH (propionic acid) ...................................................... (8)

In general, commercial processes for producing acetic acid by the carbonylation reaction of methanol with carbon monoxide include the Celanese process and the Cativa process, which are homogeneous catalytic reactions that are performed in the presence of liquid catalysts, and the Acetica process that is performed in the presence of a heterogeneous catalyst comprising an ion exchange resin. These processes are performed using methanol and carbon monoxide as raw materials in the presence of rhodium (Rh) or iridium (Ir) or ruthenium (Ru) as a catalyst and methyl iodide (MeI) as a co-catalyst at a temperature of 150 to 250°C and a reaction pressure of 20 to 60 atm. This reaction is a difficult process that requires the need of a reactor and peripheral devices, which are made of special materials, due to the corrosiveness of the reactants. These processes are divided by the method of using the catalyst. The initially developed acetic acid production is the Celanese process is a liquid homogeneous catalytic reaction based on a rhodium complex ion (Rh(CO)₂I₂⁻¹) consisting of rhodium (Rh), carbon monoxide and iodine ion, and is a process of producing acetic acid by dispersing carbon monoxide in a reactor so as to be brought into contact with methanol.

This process has a disadvantage in that a process for separating a catalyst from a product and reusing the separated catalyst as a liquid catalyst is complicated. Another disadvantage is that when acetic acid is to be vaporized in a flash drum or a distillation unit in order to separate the liquid catalyst from the product acetic acid, the partial pressure of carbon monoxide in the product is lowered and the rhodium ion is precipitated as a metal due to the release of carbon monoxide from the complex ion or is dissolved in the product, resulting in loss of the rhodium ion, and for this reason, the catalyst should be continuously supplemented. This has the disadvantage of imposing a considerable burden on the production cost. Accordingly, many researchers have conducted studies on inorganic carriers or polymers having on the surface thereof a ligand capable of supporting the rhodium-based ion catalyst, as can be well seen in the review literature published by Christophe M et al. The rhodium catalyst that is used in the Celanese process exhibits excellent performance in the methanol carbonylation reaction, but is very expensive. For this reason, other catalysts capable of replacing the rhodium catalyst have been developed, and the Cativa (BP) process that uses iridium and ruthenium ions instead of rhodium ion was proposed. The metal prices of iridium and ruthenium that are used in this process were initially much lower than the metal price of rhodium. However, as the construction of commercial plants based on the Cativa process increased, the prices of iridium and ruthenium also rose rapidly. Accordingly, the iridium/ruthenium catalyst did not significantly help to reduce the production cost. In addition, a process for separating the catalyst as a liquid catalyst from the product is difficult, and the catalyst is lost by dissolution in the product and flows out or precipitated, thus increasing the production cost.

In order to solve the problems of the above-described liquid catalyst, Chiyoda Corp. (Japan) ionically bonded a rhodium ion to a pyridine-based ion exchange resin, and produced a complex ion with the bonded rhodium ion, and used the complex ion as a catalyst for the acetic acid production reaction. By doing so, Chiyoda Corp. excluded the conditions for separating the catalyst after the reaction, and at the same time, problems such as precipitation of the complex catalyst and dissolution and loss of the complex catalyst in the product, which are due to the reduced partial pressure of carbon monoxide. This process developed by Chiyoda Corp. is called the Acetica process. In this case, the loss of the catalyst rhodium ion used as the catalyst decreased, the rhodium ion had high activity, and the production of methyl acetate and water, which is a side reaction, was also suppressed, thereby dramatically increasing acetic acid productivity.

The original technology of the well-known Celanese process as described above is based on methanol carbonylation in the presence of a rhodium catalyst developed by Monsanto in the late 1970s, and is described well in GB Patent No. 1,233,121. In other words, it is a process that includes dissolving a rhodium compound as a liquid catalyst and methyl iodide as a co-catalyst in the solvent acetic acid, and then introducing the catalyst solution together with methanol, and subjecting the introduced methanol to a gas-liquid reaction with carbon monoxide to produce acetic acid.

This process has a problem in that because the catalyst rhodium compound has to be used after dissolution in the solvent, the content of water or methyl acetate should be increased in order to increase solubility and reactivity, and thus the production apparatus becomes larger. Another problem is that because acetic acid, water and methyl acetate should be circulated, energy for purification increases, and the generation of hydrogen and hydrogen iodide, which is a side reaction caused by an excessive amount of water, influences the corrosion of the apparatus, and thus the catalyst rhodium ion is reduced and precipitated as a metal, or other corroded metal ions adversely affect the acetic acid production reaction. For these reasons, a recovery process for recycling this liquid rhodium catalyst and a regeneration process for maintaining the activity of the catalyst are required, thus increasing the production cost and the operating cost.

In an attempt to solve these problems that are caused by the liquid rhodium catalyst, European Patent No. 0277824 and Korean Patent Application Publication No. 10-1989-0012932 disclose an invention relating to the synthesis of acetic acid in the presence of a rhodium metal supported on an insoluble polymer that is a basic ion-exchange resin having an N-oxide or quaternary pyridine group.

Regarding the basic ion-exchange resin, European Patent No. 0277824 and Korean Patent Application No. 10-1989-0012932, both assigned to Reilly Industries, Inc., disclose an invention in which a rhodium ion immobilized on a polymer (basic ion-exchange resin) obtained by copolymerizing 4-vinylpyridine with divinylbenzene is used as a heterogeneous catalyst for acetic acid production.

In this copolymer (basic ion-exchange resin) of 4-vinylpyridine and divinylbenzene, which is used as a support for the rhodium ion, the copolymerization content of divinylbenzene significantly affects not only the temperature of use and solvent resistance of the copolymer, but also the ionic bonding of the rhodium ion. Furthermore, since this copolymer acts as a spherical rhodium-based ion catalyst support having a particle size of less than 1 mm, the reaction process that produces acetic acid by the methanol carbonylation process should be operated in a gas-liquid-solid slurry reaction system that is a suspension bubble column in which the catalyst flows. In this case, there is a disadvantage in that either a device capable of filtering the catalyst in order to separate the catalyst from the product acetic acid or a precipitation tank for preventing the catalyst from flowing out of the reactor should be disposed in the reactor. Even if a device capable of solving this problem is disposed in the slurry reactor, the catalyst particles collide with one another while the reactant gas carbon monoxide flows upward because of the characteristics of the slurry reactor, or the rhodium complex ion and the copolymer are detached by friction or crushed to generate powder, and thus it is difficult to avoid loss of the catalyst comprising the rhodium complex ion and the vinylpyridine-divinylbenzene copolymer (basic ion-exchange resin). In addition, operation of the reactor is stopped and the reactor is left to stand for a long period of time, this catalyst is precipitated and charged, and for this reason, high initial pressure is applied to carbon monoxide that is used to disperse and flow this catalyst so as to induce a normal acetic acid production reaction in the next operation, and the catalyst does not easily flow, thus causing many problems in operation of the slurry reactor.

In other words, the conventional polymer obtained by copolymerizing vinylpyridine with divinylbenzene is a general ion-exchange resin comprising spherical particles having a particle size of 1 mm or less. If the molar ratio of vinylpyridine is more than 75%, the amount of rhodium complex ion supported increases, but there are problems in that the three-dimensional bonding by divinylbenzene is weak, and thus the catalyst particles are easily attacked by the raw materials and the product at the acetic acid production process temperature and pressure so that these particles become smaller in size and powdered to significantly reduce their durability. If the molar ratio of vinylpyridine is less than 75%, the durability of the catalyst particles increases, but there are problems in that the amount of rhodium complex ion supported decreases because the content of pyridine is low, and thus the reactivity of the catalyst decreases and the loss of the rhodium ion also increases. Another problem is that because the basic copolymer made as described above is spherical particles having a particle size distribution ranging from 0.5 mm to 1.0 mm, the acetic acid production reaction should be performed using a catalyst support in a suspension bubble column reactor. In addition, in order to separate the catalyst from the product after the reaction, a filter device should be disposed in the reactor, or a device for precipitation should be disposed in the reactor so that a flow-free stable fluid layer will be formed so as to precipitate the catalyst and prevent the catalyst from being discharged while only the product will be discharged. In addition, in the suspension bubble column, the rhodium complex ion/ion exchange resin catalyst particles collide or rub against one another during flowing of the reactants, and thus are lost as powder, resulting in loss of the rhodium ion. Furthermore, the polymer used as the catalyst support also flows out of the reactor. Accordingly, the cycle for supplementing or replacing the catalyst is shortened, thus increasing the production cost.

Materials that can be present in a solution state during the methanol carbonylation reaction include methanol and carbon monoxide, which are raw materials, methyl iodide which is a co-catalyst, acetic acid and methyl acetate, as well as dimethylether, hydrogen iodide, water, hydrogen, carbonic acid gas, and the like, which are by-products. Accordingly, these materials include a strong solvent and corrosive reactants. Accordingly, supports, which can adsorb and support a rhodium complex ion without undergoing dissolution and corrosion under such conditions, include activated carbon, alumina, silica, clay, ceramic and the like. US Patent No. 3,769,329 mentions that a rhodium-based catalyst (Rh(CO)₂I₂⁻) may be used in a state in which it is dispersed and adsorbed on the ceramic support as described. However, the rhodium-based catalyst was not commercialized because loss of the rhodium ion could not be prevented by adsorption.

### SUMMARY

Accordingly, the present invention has been made keeping in mind the above problems occurring in the prior art, and an object of the present invention is to provide an improved novel heterogeneous catalyst for producing acetic acid by carbonylation of methanol, in which the novel heterogeneous catalyst is obtained by chemically bonding vinylpyridine capable of supporting a rhodium complex ion to a fluoropolymer support having strong solvent resistance, and is not a fluidized catalyst, but is a fixed-bed catalyst that can efficiently perform a gas-liquid catalytic reaction, because the shape of the catalyst can be freely changed unlike the shape of a pyridine-based ion-exchange resin type support composed of a vinylpyridine/divinylbenzene copolymer which is used in the Acetica process.

To achieve the above object, the present invention provides a heterogeneous catalyst for producing acetic acid by carbonylation of methanol, wherein a rhodium complex ion is ionically bonded to an insoluble catalyst support, and the insoluble catalyst support includes a fluoropolymer having a quaternary pyridine radical alone or in combination with an acetate radical grafted on the surface thereof.

In the heterogeneous catalyst for producing acetic acid by carbonylation of methanol according to the present invention, grafting the pyridine radical as a ligand on the surface of the fluoropolymer is preferably performed by diluting vinylpyridine in a solvent to a concentration of 20 wt% to 70 wt%, adding Mohr's salt as a polymerization inhibitor thereto, and irradiating cobalt gamma rays.

In the heterogeneous catalyst for producing acetic acid by carbonylation of methanol according to the present invention, grafting the pyridine radical and the acetate group as a ligand to the surface of the fluoropolymer is preferably performed by mixing more than 0 mol% but not more than 35 mol% of vinyl acetate with vinylpyridine to obtain a vinylpyridine/vinyl acetate mixture, diluting the vinylpyridine/vinyl acetate mixture in a solvent to a concentration of 20 wt% to 70 wt%, adding Mohr's salt as a polymerization inhibitor thereto, and irradiating cobalt gamma rays.

In the heterogeneous catalyst for producing acetic acid by carbonylation of methanol according to the present invention, the fluoropolymer for grafting is preferably any one selected from the group consisting of PTFE (polytetrafluoroethylene), PFA (perfluoroalkoxy resin), and a mixture thereof.

In the heterogeneous catalyst for producing acetic acid by carbonylation of methanol according to the present invention, the polymer is preferably shaped into any one or more selected from the group consisting of a sphere shape, a Raschig ring shape, a Pall ring shape, a tri-pack shape, a tellerette shape, and a saddle ring shape.

In the heterogeneous catalyst for producing acetic acid by carbonylation of methanol according to the present invention, the Mohr's salt is added in an amount of 0.1 wt% to 5 wt%.

In the heterogeneous catalyst for producing acetic acid by carbonylation of methanol according to the present invention, divinylbenzene as a crosslinking agent is preferably used in an amount of 0.1 mol% to 5 mol% based on the ligand compound in grafting of the ligand on the surface of the fluoropolymer in order to increase the ligand grafted on the surface of the fluoropolymer.

In the heterogeneous catalyst for producing acetic acid by carbonylation of methanol according to the present invention, cobalt gamma rays are preferably irradiated with a total dose of 40 kGy to 150 kGy such that the ligand compound is radically grafted onto the surface of the fluoropolymer.

In the heterogeneous catalyst for producing acetic acid by carbonylation of methanol according to the present invention, one obtained by shaping a composition including the fluoropolymer and any one or more selected from the group consisting of alumina, clay, carbon, and silica is preferably used as the support.

In the heterogeneous catalyst for producing acetic acid by carbonylation of methanol according to the present invention, one obtained by applying the fluoropolymer on the shaped surface of any one selected from the group consisting of alumina, clay, carbon, and silica is preferably used as the support.

### BRIEF DESCRIPTION OF THE DRAWINGS

The above and other objects, features and advantages of the present invention will be more clearly understood from the following detailed description taken in conjunction with the accompanying drawings, in which:
Fig. 1 schematically illustrates methods of grafting ligands on the surface of a fluoropolymer; and
Fig. 2 schematically illustrates heterogeneous catalysts for producing acetic acid by carbonylation of methanol.

### DETAILED DESCRIPTION

It is an object of the present invention is to provide an improved novel heterogeneous catalyst for producing acetic acid by carbonylation of methanol, in which the novel heterogeneous catalyst is obtained by chemically bonding vinylpyridine capable of supporting a rhodium complex ion to a fluoropolymer support having strong solvent resistance, and is not a fluidized catalyst, but is a fixed-bed catalyst that can efficiently perform a gas-liquid catalytic reaction, because the shape of the catalyst can be freely changed unlike the shape of a pyridine-based ion-exchange resin type support composed of a vinylpyridine/divinylbenzene copolymer which is used in the Acetica process.

In order to make this new conceptual catalyst, the present inventors have conducted studies on a method of bonding a vinylpyridine monomer to the surface of a fluoropolymer, ceramic or carbon support so as to expose a pyridine ligand on the surface. It is currently difficult to apply a conventional technology in order to this method to a ceramic or carbon support having strong solvent resistance. Accordingly, a method designed in the present invention is as follows.

Durable polymers, which are not corroded and dissolved in the presence of carbon monoxide, methanol, acetic acid, methyl iodide as a co-catalyst, and methyl acetate and hydrogen iodide as by-products under reaction conditions, are Teflon-based polymers having strong temperature resistance and solvent resistance. Among such Teflon-based polymers, polymers proven to be useable as supports in temperature resistance and solvent resistance tests are summarized in Table 1 below.

As shown in Table 1, co-polymers containing hydrogen attached to carbon atoms cannot be used due to their weak temperature resistance and solvent resistance under most of acetic acid synthesis reaction conditions. Teflon-based polymers having temperature resistance and solvent resistance under methanol carbonylation conditions are PTFE, PFA and FEP. Accordingly, in order to use such Teflon polymers as supports for rhodium complex ions, a method of grafting pyridine on the surface of a fluoropolymer by chemical bonding so as to expose the pyridine ligand on the surface may be used, and this grafted fluoropolymer may be used as a catalyst support in acetic acid synthesis. If a rhodium complex ion can be attached to the support surface by this method, a catalyst support can be prepared by bonding pyridine to the surface of a fluoropolymer having various shapes advantageous for the design of chemical engineering reactor systems, and thus a fixed-bed bubble reactor can be easily designed. In addition, a special device for catalyst separation is not required, and thus the device manufacturing cost can be saved, the operating cost can be reduced due to process simplification, and productivity can be greatly increased. The present inventors have envisaged a method of irradiating gamma rays to a vinylpyridine monomer solution on fluoroplastic fillers suitable for engineering design, for example, Raschig rings, Pall rings, tri-pack, tellerette, saddle rings or the like, so as to induce the radical reaction of the vinylpyridine monomer with the surface of the Teflon polymer to graft a pyridine on the filler surface to make a pyridine ligand which is used as a support for a rhodium ion. Hereinafter, a method for making a rhodium complex ion catalyst support for methanol carbonylation will be described in detail, and the catalyst prepared by this method solves the problems of conventional processes of producing acetic acid by methanol carbonylation.

**Table 1**

| Name | Chemical Name | Structure | Melting point (°C) | Solubility of reactant |
|---|---|---|---|---|
| ETFE | Tetrafluoroethylene-ethylene copolymer | -(CH₂CH₂)ₙ-(CF₂CF₂)ₘ- | 267 | x |
| PFA | Tetrafluoroethylene-Perfluorovinylether copolymer | -(CF₂CF₂)ₙ-(CF(OC₃F₇)CF₂)ₘ- | 306 | ○ |
| FEP | Tetrafluoroethylene-Hexafluoropropylene copolymer | -(CF₂CF₂)ₙ-(CF(CF₃)CF₂)ₘ- | 260 | Δ |
| PTFE | Poly Tetrafluoroethylene | -(CF₂CF₂)ₙ- | 327 | ⊚ |
| PVDF | Poly Vinylidene Fluoride | -(CH₂CF₂)ₙ- | 171 | x |
| ECTFE | Ethylene Chlorotrifluoroethylene Copolymer | -(CF₂CClF)ₙ-(CH₂CH₂)ₘ- | 238 | x |

As shown in Table 1 above, the durability of fluoropolymers in an acetic acid production system was best with PTFE, followed by PFA and FEP. Accordingly, in the present invention, catalyst supports made of PTFE and PFA were prepared and used. According to the method disclosed in the present invention, a ligand compound may also be grafted on a Raschig ring such as a spherical shape, which comprises the above-described fluoropolymer in combination with a ceramic material such as carbon, alumina, clay or the like, and the Raschig ring grafted with the ligand compound may be used as a catalyst support for a rhodium complex ion. Accordingly, the scope of the present invention is not limited only to a pure fluoropolymer support.

To achieve the above-described object, the present invention will be described in detail.

As disclosed in European Patent No. 0277824 and US Patent No. 5,364,963, an ion-exchange resin composed of a copolymer of vinylpyridine and divinylbenzene is used as a catalyst support capable of supporting a rhodium complex ion which is used to produce acetic acid by carbonylation of methanol. This resin is composed of small spherical particles having an average diameter of 0.5 to 0.7 mm or 1 mm or less. When these particles are used as a catalyst in a gas-liquid reaction, there are many problems as described above in that it is difficult to calculate mass transfer coefficient (kLa) with a reaction in the design of a reactor, and in that these particles have weak temperature resistance and solvent resistance, collide or rub against one another in the reactor to form fine powder so as to reduce their durability. As one problem, the reaction rate can be increased only when carbon monoxide that is gaseous in a gas-liquid reaction moves upward while being present as uniform and small bubbles in a liquid reactant. However, when a catalyst composed of small ion-exchange resin particles is distributed in liquid, it is very difficult to predict the behavior of bubbles moving upward in a reactant slurry. The gaseous phase that is carbon monoxide is made into small bubbles on a dispersion plate, and these small bubbles are combined into large bubbles during moving upward and move upward rapidly. Accordingly, the gas-liquid surface area is smaller than that in upward movement of relatively small bubbles, and thus mass transfer rate with a reaction is low. Accordingly, it is not easy to calculate, estimate or measure engineering data on mass transfer for the design of a bubble column reactor packed with small ion-exchange resin particles.

A ligand for immobilizing a rhodium complex ion is a quaternary ammonium ligand, and a pyridine ligand is well known as a pyridine ligand capable of immobilizing a rhodium complex catalyst for a reaction for producing acetic acid by methanol carbonylation. In the present invention, vinylpyridine is selected as a ligand for immobilizing a rhodium complex catalyst and is radically grafted onto the surface of a fluoropolymer support, which shows excellent chemical resistance and heat resistance under methanol carbonylation conditions for acetic acid synthesis, by Co γ-ray irradiation, so as to form a highly reactive radical, and the grafted fluoropolymer support is used as a support for a rhodium complex catalyst. Alternatively, a mixture of vinylpyridine and vinyl acetate is grafted on the surface of the fluoropolymer support, and the grafted support is used as a support for a rhodium complex catalyst.

Turmanova et al. and Beom-Seok Ko et al. published literature showing that ligands such as vinylpyridine are grafted on various polymer films in order to make media that adsorb heavy metals or that are used as ion conductive ion membranes. The support used in this study is a fluoropolymer and is immersed in a grafting solution such as vinylpyridine by use of a packing material that is used in chemical engineering unit operations such as distillation or adsorption/desorption. Next, cobalt gamma-rays are irradiated to the solution so as to form radicals on the fluoropolymer and the vinylpyridine, and the formed radicals react with each other such that the vinylpyridine is grafted on the surface of the fluoropolymer. The vinylpyridine-grafted fluoropolymer is used as a ligand for immobilizing a rhodium complex ion.

In this case, it is possible to solve the above-described problems occurring in a spherical ion-exchange resin powder catalyst support composed of a crosslinked copolymer of vinylpyridine and divinylbenzene as disclosed in US Patent No. 5,364,963. In addition, a rhodium complex ion catalyst support can be easily prepared without performing a polymerization process for producing an ion-exchange resin.

Hereinafter, a method of grafting a ligand on the surface of a Raschig ring, and an acetic acid synthesis reaction employing the ligand-grafted Raschig ring as a catalyst support will be described, and the present invention will be described in detail through specific examples.

### Method of Grafting Ligand on Surface of Raschig Ring

Each of various Raschig rings composed of fluoropolymers is washed under reflux with methanol for 3 hours or more, dried in a vacuum dryer at 80°C for 12 hours or more, and then placed in a nitrogen atmosphere glove box in which oxygen and water is 1 ppm or less. In addition, in the glove box, 4-VP and a crosslinking agent such as acrylic acid or divinylbenzene are mixed at a predetermined molar ratio, and the mixture is dissolved in a solvent such as methanol to a predetermined concentration (wt%), and then Mohr's salt as a polymerization inhibitor is added thereto, thereby preparing a grafting solution. Each of the Raschig rings is filled in a 50cc glass vial up to a level of 30cc, and then the prepared grafting solution is filled in the glass vial up to the indicated level.

The amount of the grafting solution is adjusted such that the Raschig ring does not protrude from the solution surface. A sufficient amount of the grafting solution may also be filled such that the Raschig ring does not protrude from the solution surface, but this is not very helpful for grafting and it only wastes the solution. The glass vial filled with the Raschig ring, the grafting solution and nitrogen is capped tightly, taken out from the glove box, and irradiated with cobalt gamma-rays. Due to cobalt gamma-ray irradiation, Raschig ring and vinyl monomers are activated into molecules having highly reactive radicals, and the activated vinyl monomer is chemically bonded (i.e., grafted) to the surface of the fluoropolymer by a radical reaction.

After cobalt gamma-rays are irradiated at a controlled dose rate (kGy/hr) for 15 hours, unreacted grafting solution is removed, and a sufficient amount of 1N-HCl solution is added to the Raschig ring grafted with the vinyl monomer, and is boiled at about 100°C (boiling point) for 6 hours in an Erlenmeyer flask equipped with a reflux condenser. Next, the HCl solution was discarded, and the Raschig ring is washed with distilled water until the pH of the washing solution reaches 5 or more. Next, the Raschig ring is added to methanol, is heated to the boiling point of methanol in an Erlenmeyer flask equipped with a reflux condenser, and is maintained at that temperature for 3 hours to wash out unreacted vinyl monomer or ungrafted homopolymer attached thereto.

The vinylpyridine-grafted Raschig ring washed as described above is dried in a vacuum oven at a temperature of 60°C or less for 12 hours, and is used as a support for a rhodium complex ion. Table 2 below shows the kinds and physical properties of Raschig rings used in the experiment, and the amount of grafting according to the dose of cobalt gamma rays irradiated to the Raschig ring used as a catalyst support in the Example is expressed as DOG (Degree of Grafting)%. DOG% is calculated using the following equation: $DOG \left(%\right) = \left[\left(Wg-Wo\right)/Wo\right] \times 100$ Wg=Weight of Grafted Raschig ring, Wo=Weight of Original Raschig ring

**Table 2**

| Table 2: Samples of Raschig Ring | | | | | | | |
|---|---|---|---|---|---|---|---|
| Sample Name | Raschig Ring Polymer | Normal Size, mm | Wall Thickness, mm | Outer Diameter and Length mm | Approximate No. of elements per Liter | Approximate weight per Liter, g | Percent Void Volume |
| FEP12.5 | FEP | 12.5 | 1.5 | 12.5 | 338 | 506.11 | 75.08 |
| PFA08.0 | PFA | 8.0 | 1.0 | 8.0 | 1574 | 655.99 | 70.79 |
| PTFE10.0 | PTFE | 10.0 | 1.0 | 10.0 | 723 | 478.96 | 76.58 |
| PTFE08.0 | PTFE | 8.0 | 1.0 | 8.0 | 1574 | 609.78 | 70.79 |
| PTFE06.0 | PTFE | 6.0 | 1.0 | 6.0 | 3766 | 749.96 | 64.24 |

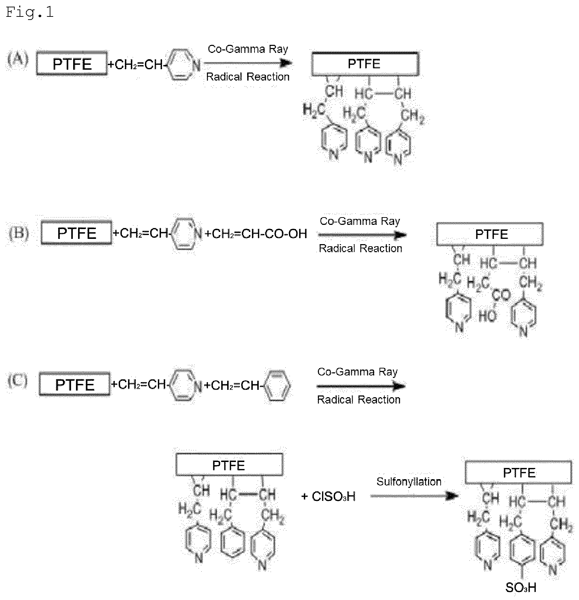

There are methods in which 4-vinylpyridine and 2-vinylpyridine, which are vinyl monomers for alkaline ligands, acrylic acid for an acidic ligand, and styrene, are grafted on fluoropolymers, and then sulfonic acid is introduced into the benzene ring by a sulfonation reaction. Fig. 1 schematically illustrates methods of grafting ligands on the surface of fluoropolymers.

### Acetic Acid Synthesis Reaction Employing Ligand-Grafted Raschig Ring as Catalyst Support

In the acetic acid synthesis reaction by methanol carbonylation, as disclosed in US Patent No. 5,364,963, a rhodium-based heterogeneous catalyst is prepared using a rhodium salt such as rhodium chloride (RhCl₃), and a support composed of a copolymer of vinylpyridine and divinylbenzene, that is, an ion-exchange resin (Reilex 425, etc.). Then, the prepared heterogeneous catalyst, acetic acid as a solvent, methanol, methyl iodide (CH₃I) as a co-catalyst, and water (H₂O), are mixed at a suitable ratio and placed in a reactor, and air is replaced with carbon monoxide. When the reaction temperature is elevated to 170°C to 200°C and the pressure of the reactor is increased to 20-50 atm, a methanol carbonylation reaction occurs to produce acetic acid.

The concentrations of methanol (which is introduced as a liquid reactant), methyl iodide and water may influence the partial pressure of the reactant at an initial reaction temperature, and thus greatly influence the reaction rate by influencing the partial pressure of carbon monoxide in total pressure. Accordingly, controlling the concentration of an initial liquid reactant is very important, because it is influenced by the temperature-dependent partial pressure. In addition, since the partial pressure of carbon monoxide can be reduced due to methyl acetate which is produced as a by-product during progress of the reaction, the production of methyl acetate should be inhibited, or the catalyst should have a very excellent activity such that methyl acetate may immediately react with carbon monoxide by the catalyst so as to be converted into acetic acid.

The main reaction shown in reaction equation (1) in the Background Art is a highly exothermic reaction from which the heat of the reaction should be effectively removed. In addition, although there are many side reactions, the major side reaction is an equilibrium reaction in which methyl acetate and water are produced as shown in equation (2). This reaction is an equilibrium reaction that occurs between methanol as a reactant and acetic acid as a solvent, because the two chemical substances are introduced in the initial stage. Accordingly, if the acetic acid production reaction of equation (1) is dominant, acetic acid will be continuously produced to inhibit the production of methyl acetate, but if the activity of the catalyst is reduced, the production of methyl acetate will increase. As reported in patents or literatures, if the reactant contains a large amount of water, the progress rate of the reaction is increased and the production of methyl acetate is inhibited. However, as shown in equation (3), water reacts with carbon monoxide to produce hydrogen, and the reactions shown in equations (4), (5) and (6) progressively occur. Ultimately, as shown in equation (7), propionic acid which is a high-boiling-point by-product is produced to reduce the yield of acetic acid and make the purification of acetic acid difficult. For this reason, the generation of water during the reaction should be inhibited, and water in the reactant introduced needs to be accurately controlled to a suitable amount. In addition, the generation of water is undesirable, because it increases energy required to remove water in a purification process.

To measure the characteristics of the catalyst, the conversion rate of methanol and the yield of each substance were calculated using the following equations on the basis of the methanol introduced, and the loss of the rhodium complex ion catalyst was calculated by measuring rhodium in the product solution by use of an ICP analyzer. $MeOH conversion \left(%\right) = \left(MeOH \left(mol before reaction\right)-MeOH\left(mol after reaction\right)\right) * 100 / \left(MeOH\left(mol before reaction\right)\right)$ $AcOH yield \left(%\right) = \left(AcOH \left(mol after reaction\right)-AcOH \left(mol before reaction\right)\right) * 100 / \left(MeOH \left(mol before reaction\right)\right)$ $AcOMe yield \left(%\right) = \left(AcOMe\left(mol after reaction\right)-AcOMe\left(mol before reaction\right)\right) * 100 / ( \left(MeOH\left(mol before reaction\right)\right)$ ${H}_{2} O yield \left(%\right) = \left({H}_{2}O \left(mol after reaction\right)-{H}_{2}O \left(mol before reaction\right)\right) * 100 / ( \left(MeOH \left(mol before reaction\right)\right)$ $Rh Loss \left(%\right) = total amount \left(ppm\right) of rhodium dissolved in product solution / initially introduced rhodium \left(ppm\right)$

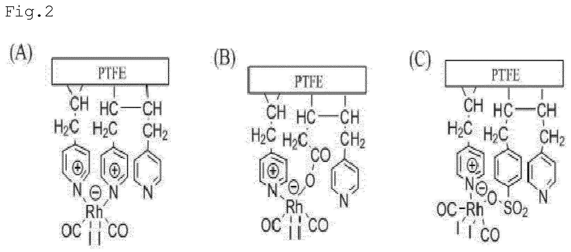

The reaction mechanism of methanol carbonylation by a rhodium complex ion catalyst is well summarized in the review report published by Christophe M. Thomas et al. Since the core compound of the rhodium complex ion catalyst is Rh(CO)₂I₂⁻, the rhodium complex ion catalyst is immobilized on a ligand compound so as not to be lost as liquid. The support that is used in this immobilization method is an alkaline ion-exchange resin prepared by copolymerizing vinylpyridine with divinylbenzene as described above, and it is well known that the Acetica process was completed by establishing a heterogeneous catalytic process. However, since this process has a number of problems as described above, the present invention has been made in order to solve these problems. In other words, as shown in Fig. 1, various ligands are grafted on the surface of a fluoropolymer, and as shown in Fig. 2, a rhodium complex ion is attached to the ligand, thereby obtaining a heterogeneous catalyst for producing acetic acid by methanol carbonylation. The heterogeneous catalyst can solve many problems. In addition, the heterogeneous catalyst makes it easy to design a fixed bed bubble reactor packed with a typical fixed bed catalyst. Furthermore, it can provide a simplified apparatus that does not require a catalyst filter unit, and it makes a simplified operation possible, indicating that the heterogeneous catalyst has excellent economic advantages. Hereinafter, description will be made of a method for preparing a fluoropolymer support grafted with a ligand, and a method of making a rhodium complex ion support catalyst using this fluoropolymer support and a rhodium salt, and then a method for producing acetic acid by methanol carbonylation in the presence of the catalyst. The Examples below illustrate specific conditions, but the scope of the present invention is not limited thereto.

A method of making a rhodium complex ion support catalyst using a fluoropolymer (i.e., Teflon) support and a rhodium salt is as follows. First, a Teflon-based support grafted with a ligand is prepared according to the above-described method and placed in an acetic acid reactor. Then, in the acetic acid reactor, acetic acid as a solvent, methanol as a reactant, and methyl iodide as a co-catalyst are mixed at a predetermined ratio, and then an acetic acid synthesis reaction is performed in the presence of a rhodium salt corresponding to 400 to 1000 ppm based on the total weight of the solution. The method for making the rhodium complex ion support catalyst is as follows. A Teflon Raschig ring grafted with a ligand is placed in a reactor, and a rhodium salt, for example, rhodium chloride (RhCl₃) that is most easily available, is placed in the reactor. As the rhodium catalyst, any rhodium compound that dissolves in a reactant may be used. In addition, for an acetic acid synthesis reaction, methanol, methyl iodide, and acetic acid as a solvent, are placed in the reactor, and the reactor is closed. Then, air in the reactor is replaced with carbon monoxide, the reaction temperature is elevated to 190°C, and the internal pressure of the reactor is increased to 40 bar by carbon monoxide. Under such conditions, the reaction is performed for 3 hours, and the obtained catalyst is washed several times with methanol and dried in a vacuum dryer at 80°C or lower, thereby making a rhodium complex ion support catalyst as shown in Fig. 2. Because this rhodium complex ion support catalyst is made under the same conditions as those of the acetic acid synthesis reaction by methanol carbonylation, the first reaction performed as described above is a catalyst preparation reaction. In the next reactions, methanol carbonylation is performed in the presence of the prepared rhodium complex ion support catalyst so as to produce acetic acid while the activity of the catalyst is measured. Measurement of the activity of the catalyst in the present invention merely corresponds to the conditions described herein, and it is already known that the activity of the catalyst can become better under other conditions where gas-liquid contact is better. Accordingly, it should be understood that the measured values are relative values used to compare between rhodium complex ion support catalysts.

### Example 1

A variety of Teflon Raschig rings (13 Raschig rings (12.5 mm (10 mm inner diameter); 19.35 g), 28 Raschig rings (10 mm (8 mm inner diameter); 19.02 g), and 60 Raschig rings 8 mm (6 mm inner diameter); 25.48 g)) were washed with methanol for 6 hours in the respective Soxhlet apparatuses, and then dried in a vacuum dryer at 80°C for 8 hours, and placed in a nitrogen atmosphere glove box in which oxygen and water were controlled to 1 ppm or less. For preparation of grafting solutions, 4-vinylpyridine (4-VP, 95%, 100 ppm hydroquinone inhibitor; purchased from Sigma-Aldrich) was vacuum-distilled to remove the polymerization inhibitor. The 4-VP purified as described above and the unpurified 4-VP were dissolved in methanol (MeOH, 99.99% HPLC, Sigma-Aldrich) as a solvent, thereby preparing 40 wt% 4-VP/methanol solutions in the nitrogen atmosphere glove box. For homopolymer inhibition, Mohr's salt (99%, Sigma-Aldrich) was added to the solutions to prepare 4-VP/methanol grafting solutions containing 0.0 wt% to 1.5 wt% of Mohr's salt, which were used as 4-VP grafting solutions. 50-cc glass vials and airtight caps were washed with methanol and dried, after which about 30 cc of each Raschig ring whose weight (Wo) was accurately measured was placed in each glass vial which was then placed in the nitrogen atmosphere glove box. Next, each glass vial was filled with each 4-VP/methanol solution prepared in the glove box such that the Raschig ring was immersed in the solution. Next, each glass vial was capped, taken out from the glove box, and irradiated with Co gamma rays. Each glass vial was irradiated at a dose rate of 3 kGy/hr to 5 kGy/hr for 15 hours, and then each Raschig ring was recovered from the grafting solution and boiled with 500 cc of 1N hydrochloric acid solution for 3 hours in a flask equipped with a reflux condenser so as to wash out the Mohr's salt and the homopolymer. Next, each Raschig ring was washed sufficiently with distilled water until a neutral pH was reached, after which it was boiled with methanol for about 3 hours in a flask equipped with a reflux condenser as described above so as to wash out the monomer and homopolymer attached to the Raschig ring surface grafted with 4-VP. Next, each Raschig ring was dried, and then the weight (Wg) thereof was measured. Based on the weights, the DOG (%) of the ligand compound was calculated, and the results are shown in Tables 3 and 4 below.

**Table 3**

| Example 1: DOG% according to the material of Teflon Raschig ring and conditions for Co-γ ray irradiation | | | | | | | | | | |
|---|---|---|---|---|---|---|---|---|---|---|
| No. | Sample Name | Raschig ring polymer | Raschig ring, mm | Grafting chemical | Inhibitor ○, x | Mohr's salt, % | Grafting chemical Conc. % | Co γ-ray radiation, kGy/hr | Raschig ring, weight, Wo, g | DOG, % |
| 1 | FEP12.5-4VPI-40-3KG/15 | FEP | 12.5 | 4-VP | ○ | 0.0 | 40 | 3-15 | 19.3526 | 0.49 |
| 2 | FEP12.5-4VPIM1.5-40-3KG/15 | FEP | 12.5 | 4-VP | ○ | 1.5 | 40 | 3-15 | 19.3550 | 0.55 |
| 3 | PTFE10-4VPI-40-3KG/15 | PTFE | 10.0 | 4-VP | ○ | 0.0 | 40 | 3-15 | 19.0262 | 2.37 |
| 4 | PTFE10-4VPIM1.5-40-3KG/15 | PTFE | 10.0 | 4-VP | ○ | 1.5 | 40 | 3-15 | 18.7107 | 2.49 |
| 5 | PFA08-4VPI-40-3KG/15 | PFA | 8.0 | 4-VP | ○ | 0.0 | 40 | 3-15 | 25.2190 | 1.32 |
| 6 | PFA08-4VPIM1.5-40-3KG/15 | PFA | 8.0 | 4-VP | ○ | 1.5 | 40 | 3-15 | 25.4809 | 1.36 |
| 7 | FEP12.5-4VEM1.5-40-3KG/15 | FEP | 12.5 | 4-VP | x | 1.5 | 40 | 3-15 | 19.7166 | 0.53 |
| 8 | PTFE10-4VEM1.5-40-3KG/15 | PTFE | 10.0 | 4-VP | x | 1.5 | 40 | 3-15 | 19.0770 | 2.28 |
| 9 | PFA08-4VPM1.5-40-3KG/15 | PFA | 8.0 | 4-VP | x | 1.5 | 40 | 3-15 | 23.9623 | 1.64 |
| 10 | PTFE10-4VPIM0.5-40-4KG/15 | PTFE | 6.0 | 4-VP | ○ | 0.5 | 40 | 4-15 | 17.9600 | 2.88 |
| 11 | PTFE10-4VPIM1.0-40-4KG/15 | PTFE | 6.0 | 4-VP | ○ | 1.0 | 40 | 4-15 | 17.3988 | 2.86 |
| 12 | PTFE10-4VPIM1.5-40-4KG/15 | PTFE | 6.0 | 4-VP | ○ | 1.5 | 40 | 4-15 | 18.0438 | 2.81 |
| 13 | PTFE10-4VPIM0.5-60-4KG/15 | PTFE | 6.0 | 4-VP | ○ | 0.5 | 60 | 4-15 | 18.1108 | 2.27 |

**Table 4**

| | | | | | | | | | | |
|---|---|---|---|---|---|---|---|---|---|---|
| 14 | PTFE10-4VPIM1.0 -60-4KG/15 | PTFE | 6.0 | 4-VP | ○ | 1.0 | 60 | 4-15 | 17.6821 | 2.24 |
| 15 | PTFE10-4VPIM1.5 -60-4KG/15 | PTFE | 6.0 | 4-VP | ○ | 1.5 | 60 | 4-15 | 18.0470 | 2.80 |
| 16 | PTFE10-4VPIM0.5 -40-5KG/15 | PTFE | 6.0 | 4-VP | ○ | 0.5 | 40 | 5-15 | 17.4447 | 3.33 |
| 17 | PTFE10-4VPIM1.0 -40-5KG/15 | PTFE | 6.0 | 4-VP | ○ | 1.0 | 40 | 5-15 | 17.7843 | 3.10 |
| 18 | PTFE10-4VPIM1.5 -40-5KG/15 | PTFE | 6.0 | 4-VP | ○ | 1.5 | 40 | 5-15 | 18.4055 | 3.13 |
| 19 | PTFE10-4VPIM0.5 -60-5KG/15 | PTFE | 6.0 | 4-VP | ○ | 0.5 | 60 | 5-15 | 18.6520 | 2.18 |
| 20 | PTFE10-4VPIM1.0 -60-5KG/15 | PTFE | 6.0 | 4-VP | ○ | 1.0 | 60 | 5-15 | 17.4324 | 2.17 |
| 21 | PTFE10-4VPIM1.5 -60-5KG/15 | PTFE | 6.0 | 4-VP | ○ | 1.5 | 60 | 5-15 | 17.8885 | 2.30 |

### Example 2

According to the same method as described in Example 1, 100 PTFE Raschig rings (about 19.5 g; 6 mm outer diameter, 4 mm inner diameter) were washed with methanol for 6 hours in the respective Soxhlet apparatuses, and then dried in a vacuum dryer at 80°C for 8 hours, and placed in the nitrogen atmosphere glove box. For preparation of grafting solutions, 4-vinylpyridine (4-VP, 95%, 100 ppm hydroquinone inhibitor; purchased from Sigma-Aldrich) was used without purification and dissolved in methanol (MeOH, 99.9% HPLC, Sigma-Aldrich) as a solvent, thereby preparing 30 wt%, 40 wt%, 50 wt% and 60 wt% 4-VP/methanol solutions in a nitrogen atmosphere glove box.

For homopolymer inhibition, Mohr's salt (99%, Sigma-Aldrich) was added to the solutions to prepare 4-VP/methanol grafting solutions containing 0 wt% to 0.5 wt% of Mohr's salt, which were used as 4-VP grafting solutions. 50-cc glass vials and airtight caps were washed with methanol and dried, after which about 30 cc of each Raschig ring whose weight (Wo) was accurately measured was placed in each glass vial which was then placed in the nitrogen atmosphere glove box. Next, each glass vial was filled with each 4-VP/methanol solution prepared in the glove box such that the Raschig ring was immersed in the solution. Next, each glass vial was capped, taken out from the glove box, and irradiated with Co gamma rays. Each glass vial was irradiated at a dose rate of 5 kGy/hr, 6 kGy/hr or 7 kGy/hr for 15 hours, and then each Raschig ring was recovered from the grafting solution and boiled with 1N hydrochloric acid solution for 3 hours in a flask equipped with a reflux condenser so as to wash out the Mohr's salt and the homopolymer. Next, each Raschig ring was washed sufficiently with distilled water until a neutral pH was reached, after which it was boiled with methanol for about 3 hours in a flask equipped with a reflux condenser as described above so as to wash out the monomer and homopolymer attached to the Raschig ring surface grafted with 4-VP. Next, each Raschig ring was dried, and then the weight (Wg) thereof was measured. Based on the weights, the DOG (%) of the ligand compound was calculated, and the results are shown in Tables 5, 6 and 7 below.

**Table 5**

| Example 2: DOG% of 4-VP according to conditions for Co-γ ray irradiation onto 6 mm PTFE Raschig ring | | | | | | | | | | |
|---|---|---|---|---|---|---|---|---|---|---|
| No. | Sample Name | Raschig ring polymer | Raschig ring, mm | Grafting chemical | Inhibitor ○, x | Mohr's salt, % | Grafting chemical Conc. % | Co γ-ray radiation, kGy/hr | Raschig ring, weight, Wo, g | DOG% |
| 54 | PTFE6-4VPIM0.00 -30-5KG/15 | PTFE | 6 | 4-VP | ○ | 0.0 | 30 | 5-15 | 19.4058 | 1.80 |
| 55 | PTFE6-4VPIM0.10 -30-5KG/15 | PTFE | 6 | 4-VP | ○ | 0.10 | 30 | 5-15 | 19.3975 | 1.90 |
| 56 | PTFE6-4VPIM0.25 -30-5KG/15 | PTFE | 6 | 4-VP | ○ | 0.25 | 30 | 5-15 | 19.4999 | 1.85 |
| 57 | PTFE6-4VPIM0.50 -30-5KG/15 | PTFE | 6 | 4-VP | ○ | 0.50 | 30 | 5-15 | 19.4459 | 1.71 |
| 58 | PTFE6-4VPIM0.00 -40-5KG/15 | PTFE | 6 | 4-VP | ○ | 0.0 | 40 | 5-15 | 19.3378 | 3.81 |
| 59 | PTFE6-4VPIM0.10 -40-5KG/15 | PTFE | 6 | 4-VP | ○ | 0.10 | 40 | 5-15 | 19.4231 | 3.90 |
| 60 | PTFE6-4VPIM0.25 -40-5KG/15 | PTFE | 6 | 4-VP | ○ | 0.25 | 40 | 5-15 | 19.4682 | 3.68 |
| 61 | PTFE6-4VPIM0.50 -40-5KG/15 | PTFE | 6 | 4-VP | ○ | 0.50 | 40 | 5-15 | 19.4184 | 3.70 |
| 62 | PTFE6-4VPIM0.00 -50-5KG/15 | PTFE | 6 | 4-VP | ○ | 0.0 | 50 | 5-15 | 19.3777 | 4.48 |
| 63 | PTFE6-4VPIM0.10 -50-5KG/15 | PTFE | 6 | 4-VP | ○ | 0.10 | 50 | 5-15 | 19.4038 | 4.99 |
| 64 | PTFE6-4VPIM0.25 -50-5KG/15 | PTFE | 6 | 4-VP | ○ | 0.25 | 50 | 5-15 | 19.3336 | 5.82 |
| 65 | PTFE6-4VPIM0.50 -50-5KG/15 | PTFE | 6 | 4-VP | ○ | 0.50 | 50 | 5-15 | 19.4075 | 4.95 |
| 66 | PTFE6-4VPIM0.00 -60-5KG/15 | PTFE | 6 | 4-VP | ○ | 0.0 | 60 | 5-15 | 19.3908 | 4.24 |

**Table 6**

| | | | | | | | | | | |
|---|---|---|---|---|---|---|---|---|---|---|
| 67 | PTFE6-4VPIM0.10-60-5KG/15 | PTFE | 6 | 4-VP | ○ | 0.10 | 60 | 5-15 | 19.3878 | 3.99 |
| 68 | PTFE6-4VPIM0.25-60-5KG/15 | PTFE | 6 | 4-VP | ○ | 0.25 | 60 | 5-15 | 19.3074 | 4.50 |
| 69 | PTFE6-4VPIM0.50-60-5KG/15 | PTFE | 6 | 4-VP | ○ | 0.50 | 60 | 5-15 | 19.3853 | 4.21 |
| 70 | PTFE6-4VPIM0.00-30-6KG/15 | PTFE | 6 | 4-VP | ○ | 0.0 | 30 | 6-15 | 19.4088 | 2.30 |
| 71 | PTFE6-4VPIM0.10-30-6KG/15 | PTFE | 6 | 4-VP | ○ | 0.10 | 30 | 6-15 | 19.5798 | 2.65 |
| 72 | PTFE6-4VPIM0.25-30-6KG/15 | PTFE | 6 | 4-VP | ○ | 0.25 | 30 | 6-15 | 19.4092 | 2.58 |
| 73 | PTFE6-4VPIM0.50-30-6KG/15 | PTFE | 6 | 4-VP | ○ | 0.50 | 30 | 6-15 | 19.4346 | 2.43 |
| 74 | PTFE6-4VPIM0.00-40-6KG/15 | PTFE | 6 | 4-VP | ○ | 0.0 | 40 | 6-15 | 19.4486 | 4.65 |
| 75 | PTFE6-4VPIM0.10-40-6KG/15 | PTFE | 6 | 4-VP | ○ | 0.10 | 40 | 6-15 | 19.3696 | 5.46 |
| 76 | PTFE6-4VPIM0.25-40-6KG/15 | PTFE | 6 | 4-VP | ○ | 0.25 | 40 | 6-15 | 19.5097 | 4.31 |
| 77 | PTFE6-4VPIM0.50-40-6KG/15 | PTFE | 6 | 4-VP | ○ | 0.50 | 40 | 6-15 | 19.3818 | 4.49 |
| 78 | PTFE6-4VPIM0.00-50-6KG/15 | PTFE | 6 | 4-VP | ○ | 0.0 | 50 | 6-15 | 19.5818 | 3.73 |
| 79 | PTFE6-4VPIM0.10-50-6KG/15 | PTFE | 6 | 4-VP | ○ | 0.10 | 50 | 6-15 | 19.3584 | 5.66 |
| 80 | PTFE6-4VPIM0.25-50-6KG/15 | PTFE | 6 | 4-VP | ○ | 0.25 | 50 | 6-15 | 19.3531 | 5.93 |
| 81 | PTFE6-4VPIM0.50-50-6KG/15 | PTFE | 6 | 4-VP | ○ | 0.50 | 50 | 6-15 | 19.4200 | 5.26 |
| 82 | PTFE6-4VPIM0.00-60-6KG/15 | PTFE | 6 | 4-VP | ○ | 0.0 | 60 | 6-15 | 19.3721 | 4.84 |
| 83 | PTFE6-4VPIM0.10-60-6KG/15 | PTFE | 6 | 4-VP | ○ | 0.10 | 60 | 6-15 | 19.3672 | 5.86 |

**Table 7**

| | | | | | | | | | | |
|---|---|---|---|---|---|---|---|---|---|---|
| 84 | PTFE6-4VPIM0.25-60-6KG/15 | PTFE | 6 | 4-VP | ○ | 0.25 | 60 | 6-15 | 19.3860 | 5.69 |
| 85 | PTFE6-4VPIM0.50-60-6KG/15 | PTFE | 6 | 4-VP | ○ | 0.50 | 60 | 6-15 | 19.4195 | 5.63 |
| 86 | PTFE6-4VPIM0.00-30-7KG/15 | PTFE | 6 | 4-VP | ○ | 0.0 | 30 | 7-15 | 19.4610 | 3.00 |
| 87 | PTFE6-4VPIM0.10-30-7KG/15 | PTFE | 6 | 4-VP | ○ | 0.10 | 30 | 7-15 | 19.4276 | 3.41 |
| 88 | PTFE6-4VPIM0.25-30-7KG/15 | PTFE | 6 | 4-VP | ○ | 0.25 | 30 | 7-15 | 19.3827 | 3.10 |
| 89 | PTFE6-4VPIM0.50-30-7KG/15 | PTFE | 6 | 4-VP | ○ | 0.50 | 30 | 7-15 | 19.4098 | 3.42 |
| 90 | PTFE6-4VPIM0.00-40-7KG/15 | PTFE | 6 | 4-VP | ○ | 0.0 | 40 | 7-15 | 19.3601 | 5.22 |
| 91 | PTFE6-4VPIM0.10-40-7KG/15 | PTFE | 6 | 4-VP | ○ | 0.10 | 40 | 7-15 | 19.3578 | 5.40 |
| 92 | PTFE6-4VPIM0.25-40-7KG/15 | PTFE | 6 | 4-VP | ○ | 0.25 | 40 | 7-15 | 19.5646 | 5.35 |
| 93 | PTFE6-4VPIM0.50-40-7KG/15 | PTFE | 6 | 4-VP | ○ | 0.50 | 40 | 7-15 | 19.3995 | 5.24 |
| 94 | PTFE6-4VPIM0.00-50-7KG/15 | PTFE | 6 | 4-VP | ○ | 0.0 | 50 | 7-15 | 19.4567 | 5.33 |
| 95 | PTFE6-4VPIM0.10-50-7KG/15 | PTFE | 6 | 4-VP | ○ | 0.10 | 50 | 7-15 | 19.3668 | 5.79 |
| 96 | PTFE6-4VPIM0.25-50-7KG/15 | PTFE | 6 | 4-VP | ○ | 0.25 | 50 | 7-15 | 19.4338 | 6.00 |
| 97 | PTFE6-4VPIM0.50-50-7KG/15 | PTFE | 6 | 4-VP | ○ | 0.50 | 50 | 7-15 | 19.4370 | 7.07 |

### Example 3

According to the same method as described in Example 2, 100 PTFE Raschig rings (about 19.5 g; 6 mm outer diameter, 4 mm inner diameter) were washed with methanol for 6 hours in the respective Soxhlet apparatuses, and then dried in a vacuum dryer at 80°C for 8 hours, and placed in a nitrogen atmosphere glove box. 4-Vinylpyridine (4-VP, 95%, 100 ppm hydroquinone as inhibitor, Sigma-Aldrich) as a grafting reagent and divinylbenzene (DVB, 80%, 1000 ppm p-tert-butylcatechol as inhibitor, Sigma-Aldrich) as a crosslinking reagent were used without purification. DVB was added to 4-VP in amounts of 25 mol%, 15 mol% and 5 mol%, and then each VP/DVB mixture was dissolved in methanol (MeOH, 99.9% HPLC, Sigma-Aldrich) as a solvent to a concentration of 50 wt% to make mixture solutions which were then placed in the nitrogen atmosphere glove box. For homopolymer inhibition, Mohr's salt (99%, Sigma-Aldrich) was added to the solutions to prepare 4-VP/DVB methanol grafting solutions containing 0.1 wt% to 0.5 wt% of Mohr's salt, which were used as grafting solutions. 50-cc glass vials and airtight caps were washed with methanol and dried, after which about 30 cc of each Raschig ring whose weight (Wo) was accurately measured was placed in each glass vial which was then placed in the nitrogen atmosphere glove box. Next, each glass vial was filled with each prepared 4-VP/DVB methanol solution containing the polymerization inhibitor such that the Raschig ring was immersed in the solution. Next, each glass vial was capped, taken out from the glove box, and irradiated with Co gamma rays. Each glass vial was irradiated at a dose rate of 5 kGy/hr, 6 kGy/hr or 7 kGy/hr for 15 hours, and then each Raschig ring was recovered from the grafting solution and boiled with 1N hydrochloric acid solution for 3 hours in a flask equipped with a reflux condenser so as to wash out the Mohr's salt and the homopolymer. Next, each Raschig ring was washed sufficiently with distilled water until a neutral pH was reached, after which it was boiled with methanol for about 3 hours in a flask equipped with a reflux condenser as described above so as to wash out the monomer and homopolymer attached to the Raschig ring surface. Next, each Raschig ring was dried, and then the weight (Wg) thereof was measured. Based on the weights, the DOG (%) of the ligand compound was calculated, and the results are shown in Tables 8 and 9 below.

**Table 8**

| Example 3: DOG% of 4-VP/DVB according to conditions for Co-γ ray irradiation onto 6 mm PTFE Raschig ring | | | | | | | | |
|---|---|---|---|---|---|---|---|---|
| No. | Sample Name | 4-VP Grafting chemical, mol% | DVB Cross Linking chemical, mol% | Mohr's salt, % | Grafting Chemicals Conc. % | Co γ-ray Radiation, kGy/hr | Raschig ring, weight, Wo, g | DOG, % |
| 102 | PTFE6-4VP/DVB25-IM0.10-50-5KG/15 | 75 | 25 | 0.1 | 50 | 5-15 | 19.4656 | 0.86 |
| 103 | PTFE6-4VP/DVB25-IM0.25-50-5KG/15 | 75 | 25 | 0.25 | 50 | 5-15 | 19.4921 | 0.98 |
| 104 | PTFE6-4VP/DVB25-IM0.50-50-5KG/15 | 75 | 25 | 0.5 | 50 | 5-15 | 19.4797 | 0.91 |
| 105 | PTFE6-4VP/DVB15-IM0.10-50-5KG/15 | 85 | 15 | 0.1 | 50 | 5-15 | 19.5126 | 1.15 |
| 106 | PTFE6-4VP/DVB15-IM0.25-50-5KG/15 | 85 | 15 | 0.25 | 50 | 5-15 | 19.3991 | 1.20 |
| 107 | PTFE6-4VP/DVB15-IM0.50-50-5KG/15 | 85 | 15 | 0.5 | 50 | 5-15 | 19.4694 | 1.11 |
| 108 | PTFE6-4VP/DVB05-IM0.10-50-5KG/15 | 95 | 5 | 0.1 | 50 | 5-15 | 19.4715 | 2.43 |
| 109 | PTFE6-4VP/DVB05-IM0.25-50-5KG/15 | 95 | 5 | 0.25 | 50 | 5-15 | 19.5198 | 2.43 |
| 110 | PTFE6-4VP/DVB05-IM0.50-50-5KG/15 | 95 | 5 | 0.5 | 50 | 5-15 | 19.4725 | 2.29 |
| 129 | PTFE6-4VP/DVB25-IM0.10-50-6KG/15 | 75 | 25 | 0.1 | 50 | 6-15 | 19.4614 | 1.29 |
| 130 | PTFE6-4VP/DVB25-IM0.25-50-6KG/15 | 75 | 25 | 0.25 | 50 | 6-15 | 19.5124 | 1.24 |
| 131 | PTFE6-4VP/DVB25-IM0.50-50-6KG/15 | 75 | 25 | 0.5 | 50 | 6-15 | 19.4971 | 1.28 |
| 132 | PTFE6-4VP/DVB15-IM0.10-50-6KG/15 | 85 | 15 | 0.1 | 50 | 6-15 | 19.5188 | 1.89 |
| 133 | PTFE6-4VP/DVB15-IM0.25-50-6KG/15 | 85 | 15 | 0.25 | 50 | 6-15 | 19.5272 | 1.89 |

**Table 9**

| | | | | | | | | |
|---|---|---|---|---|---|---|---|---|
| 134 | PTFE6-4VP/DVB15-IM0.50-50-6KG/15 | 85 | 15 | 0.5 | 50 | 6-15 | 19.4770 | 1.86 |
| 135 | PTFE6-4VP/DVB05-IM0.10-50-6KG/15 | 95 | 5 | 0.1 | 50 | 6-15 | 19.4082 | 2.65 |
| 136 | PTFE6-4VP/DVB05-IM0.25-50-6KG/15 | 95 | 5 | 0.25 | 50 | 6-15 | 19.3606 | 2.55 |
| 137 | PTFE6-4VP/DVB05-IM0.50-50-6KG/15 | 95 | 5 | 0.5 | 50 | 6-15 | 19.5116 | 2.48 |
| 156 | PTFE6-4VP/DVB25-IM0.10-50-7KG/15 | 75 | 25 | 0.1 | 50 | 7-15 | 19.4986 | 1.21 |
| 157 | PTFE6-4VP/DVB25-IM0.25-50-7KG/15 | 75 | 25 | 0.25 | 50 | 7-15 | 19.3640 | 1.35 |
| 158 | PTFE6-4VP/DVB25-IM0.50-50-7KG/15 | 75 | 25 | 0.5 | 50 | 7-15 | 19.5152 | 1.26 |
| 159 | PTFE6-4VP/DVB15-IM0.10-50-7KG/15 | 85 | 15 | 0.1 | 50 | 7-15 | 19.4891 | 1.92 |
| 160 | PTFE6-4VP/DVB15-IM0.25-50-7KG/15 | 85 | 15 | 0.25 | 50 | 7-15 | 19.4903 | 2.04 |
| 161 | PTFE6-4VP/DVB15-IM0.50-50-7KG/15 | 85 | 15 | 0.5 | 50 | 7-15 | 19.4836 | 1.99 |
| 162 | PTFE6-4VP/DVB05-IM0.10-50-7KG/15 | 95 | 5 | 0.1 | 50 | 7-15 | 19.3992 | 2.47 |
| 163 | PTFE6-4VP/DVB05-IM0.25-50-7KG/15 | 95 | 5 | 0.25 | 50 | 7-15 | 19.3521 | 2.21 |
| 164 | PTFE6-4VP/DVB05-IM0.50-50-7KG/15 | 95 | 5 | 0.5 | 50 | 7-15 | 19.5103 | 2.37 |

### Example 4

According to the same method as described in Example 2, 100 Raschig rings (about 19.5 g; 6 mm outer diameter, 4 mm inner diameter) were washed with methanol for 6 hours in the respective Soxhlet apparatuses, and then dried in a vacuum dryer at 80°C for 8 hours, weighed, and placed in a nitrogen atmosphere glove box. 4-vinylpyridine (4-VP, 95%, 100 ppm hydroquinone as inhibitor, Sigma-Aldrich) as a grafting reagent for a pyridine ligand and acrylic acid (AcAc, 99%, 200 ppm MEHQ as inhibitor, Sigma-Aldrich) for an acetic acid ligand were used without purification. AcAc was added to 4-VP in amounts of 50 mol%, 35 mol% and 10 mol%, and then each 4-VP/AcAc mixture was dissolved in methanol (MeOH, 99.9% HPLC, Sigma-Aldrich) as a solvent to a concentration of 50 wt% to make 4-VP/AcAc methanol solutions in the nitrogen atmosphere glove box. For homopolymer inhibition, Mohr's salt (99%, Sigma-Aldrich) was added to each methanol solution to make 4-VP/AcAc methanol grafting solutions containing 0.1 wt% to 0.5 wt% of Mohr's salt, which were used as grafting solutions. 50-cc glass vials and airtight caps were washed with methanol, dried, and then placed in the nitrogen atmosphere glove box, and each Raschig ring was placed in each glass vial in a glove box. Next, each glass vial was filled with each prepared 4-VP/AcAc methanol grafting solution such that the Raschig ring was immersed in the solution. Next, each glass vial was capped, taken out from the glove box, and irradiated with Co gamma rays. Each glass vial was irradiated at a dose rate of 5 kGy/hr, 6 kGy/hr or 7 kGy/hr for 15 hours, and then each Raschig ring was recovered from the grafting solution and boiled with 1N hydrochloric acid solution for 3 hours in a flask equipped with a reflux condenser so as to wash out the Mohr's salt and the homopolymer. Next, each Raschig ring was washed sufficiently with distilled water until a neutral pH was reached, after which it was boiled with methanol for about 3 hours in a flask equipped with a reflux condenser as described above so as to wash out the monomer and homopolymer attached to the Raschig ring surface. Next, each Raschig ring was dried, and then the weight (Wg) thereof was measured. Based on the weights, the DOG (%) of the ligand compound was calculated, and the results are shown in Tables 10 and 11 below.

**Table 10**

| Example 4: DOG% of 4-VP/AcAc according to conditions for Co-γ ray irradiation to 6 mm PTFE Raschig ring | | | | | | | | |
|---|---|---|---|---|---|---|---|---|
| No. | Sample **Name** | 4-VP Grafting chemical, mol% | Acrylic Acid Grafting chemical, mol% | Mohr's Salt, % | Grafting Chemicals Conc. % | Co γ-ray Radiation, | Raschig ring, weight, Wo, g | DOG, % |
| 111 | PTFE6-4VP/AcAc50-IM0.10-50-5KG/15 | 50 | 50 | 0.1 | 50 | 5-15 | 19.3642 | 0.02 |
| 112 | PTFE6-4VP/AcAc50-IM0.25-50-5KG/15 | 50 | 50 | 0.25 | 50 | 5-15 | 19.4795 | 0.06 |
| 113 | PTFE6-4VP/AcAc50-IM0.50-50-5KG/15 | 50 | 50 | 0.5 | 50 | 5-15 | 19.4633 | 0.08 |
| 114 | PTFE6-4VP/AcAc35-IM0.10-50-5KG/15 | 65 | 35 | 0.1 | 50 | 5-15 | 19.5025 | 0.08 |
| 115 | PTFE6-4VP/AcAc35-IM0.25-50-5KG/15 | 65 | 35 | 0.25 | 50 | 5-15 | 19.5170 | 0.13 |
| 116 | PTFE6-4VP/AcAc35-IM0.50-50-5KG/15 | 65 | 35 | 0.5 | 50 | 5-15 | 19.5054 | 0.10 |
| 117 | PrFE6-4VP/AcAc10-IM0.10-50-5KG/15 | 90 | 10 | 0.1 | 50 | 5-15 | 19.3949 | 2.42 |
| 118 | PrFE6-4VP/AcAc10-IM0.25-50-5KG/15 | 90 | 10 | 0.25 | 50 | 5-15 | 19.5018 | 2.22 |
| 119 | PrFE6-4VP/AcAc10-IM0.50-50-5KG/15 | 90 | 10 | 0.5 | 50 | 5-15 | 19.5090 | 2.51 |
| 138 | PTFE6-4VP/AcAc50-IM0.10-50-6KG/15 | 50 | 50 | 0.1 | 50 | 6-15 | 19.3247 | 0.06 |
| 139 | PTFE6-4VP/AcAc50-IM0.25-50-6KG/15 | 50 | 50 | 0.25 | 50 | 6-15 | 19.5045 | 0.07 |
| 140 | PTFE6-4VP/AcAc50-IM0.50-50-6KG/15 | 50 | 50 | 0.5 | 50 | 6-15 | 19.4921 | 0.07 |
| 141 | PTFE6-4VP/AcAc35-IM0.10-50-6KG/15 | 65 | 35 | 0.1 | 50 | 6-15 | 19.5070 | 0.28 |
| 142 | PTFE6-4VP/AcAc35-IM0.25-50-6KG/15 | 65 | 35 | 0.25 | 50 | 6-15 | 19.4832 | 0.02 |

**Table 11**

| | | | | | | | | |
|---|---|---|---|---|---|---|---|---|
| 143 | PTFE6-4VP/AcAc35-IM0.50-50-6KG/15 | 65 | 35 | 0.5 | 50 | 6-15 | 19.3253 | 0.15 |
| 144 | PrFE6-4VP/AcAc10-IM0.10-50-6KG/15 | 90 | 10 | 0.1 | 50 | 6-15 | 19.4981 | 3.66 |
| 145 | PrFE6-4VP/AcAc10-IM0.25-50-6KG/15 | 90 | 10 | 0.25 | 50 | 6-15 | 19.4518 | 3.36 |
| 146 | PrFE6-4VP/AcAc10-IM0.50-50-6KG/15 | 90 | 10 | 0.5 | 50 | 6-15 | 19.4879 | 3.73 |
| 165 | PTFE6-4VP/AcAc50-IM0.10-50-7KG/15 | 50 | 50 | 0.1 | 50 | 7-15 | 19.3341 | 0.08 |
| 166 | PTFE6-4VP/AcAc50-IM0.25-50-7KG/15 | 50 | 50 | 0.25 | 50 | 7-15 | 19.5138 | 0.07 |
| 167 | PTFE6-4VP/AcAc50-IM0.50-50-7KG/15 | 50 | 50 | 0.5 | 50 | 7-15 | 19.3441 | 0.09 |
| 168 | PTFE6-4VP/AcAc35-IM0.10-50-7KG/15 | 65 | 35 | 0.1 | 50 | 7-15 | 19.4771 | 0.15 |
| 169 | PTFE6-4VP/AcAc35-IM0.25-50-7KG/15 | 65 | 35 | 0.25 | 50 | 7-15 | 19.5114 | 0.16 |
| 170 | PTFE6-4VP/AcAc35-IM0.50-50-7KG/15 | 65 | 35 | 0.5 | 50 | 7-15 | 19.4814 | 0.17 |
| 171 | PrFE6-4VP/AcAc10-IM0.10-50-7KG/15 | 90 | 10 | 0.1 | 50 | 7-15 | 19.3877 | 4.52 |
| 172 | PrFE6-4VP/AcAc10-IM0.25-50-7KG/15 | 90 | 10 | 0.25 | 50 | 7-15 | 19.4628 | 4.17 |
| 173 | PrFE6-4VP/AcAc10-IM0.50-50-7KG/15 | 90 | 10 | 0.5 | 50 | 7-15 | 19.4612 | 3.72 |

### Example 5

According to the same method as described in Example 2, 100 PTFE and 100 PFA Raschig rings (about 19.5 g for PTFE, about 23.5 g for PFA; 6 mm outer diameter, 4 mm inner diameter) were washed with methanol for 6 hours in the respective Soxhlet apparatuses, dried in a vacuum dryer at 80°C for 8 hours, weighed, and then placed in a nitrogen atmosphere glove box. 4-Vinylpyridine (4-VP, 95%, 100 ppm hydroquinone as inhibitor, Sigma-Aldrich) as a grafting reagent and divinylbenzene (DVB, 80%, 1000 ppm p-tert-butylcatechol as inhibitor, Sigma-Aldrich) as a crosslinking agent were used without purification. DVB was added to 4-VP in amounts of 1 mol%, 2 mol%, 3 mol% and 5 mol%, and each 4-VP/DVB mixture was dissolved in methanol (MeOH, 99.99% HPLC, Sigma-Aldrich) as a solvent to a concentration of 50 wt%, thereby preparing 4-VP/DVB methanol solutions in the nitrogen atmosphere glove box. For homopolymer inhibition, Mohr's salt (99%, Sigma-Aldrich) was added to the solutions to prepare 4-VP/DVB methanol grafting solutions containing 0.1 wt%, 0.25 wt% and 0.5 wt% of Mohr's salt, which were used as grafting solutions. 50-cc glass vials and airtight caps were washed with methanol, dried and then placed in a glove box under a nitrogen box, after which each Raschig ring in the glove box was placed in each glass vial. Next, each glass vial was filled with each prepared 4-VP/DVB methanol grafting solution such that the Raschig ring was immersed in the solution. Next, each glass vial was capped, taken out from the glove box, and irradiated with Co gamma rays. Each glass vial was irradiated at a dose rate of 7 kGy/hr, 8 or 9 for 15 hours, and then each Raschig ring was recovered from the grafting solution and boiled with 1N hydrochloric acid solution for 3 hours in a flask equipped with a reflux condenser so as to wash out the Mohr's salt and the homopolymer. Next, each Raschig ring was washed sufficiently with distilled water until a neutral pH was reached, after which it was boiled with methanol for about 3 hours in a flask equipped with a reflux condenser as described above so as to wash out the monomer and homopolymer attached to the Raschig ring surface. Next, each Raschig ring was dried, and then the weight (Wg) thereof was measured. Based on the weights, the DOG (%) of the ligand compound was calculated, and the results are shown in Tables 12, 13, 14 and 15.

**Table 12**

| Example 5: DOG% of 4-VP/DVB according to conditions for Co γ-ray irradiation to 6 mm PTFE and PFA Raschig rings | | | | | | | | |
|---|---|---|---|---|---|---|---|---|
| No. | Sample Name | 4-VP Grafting chemical, mol% | DVB Cross Linking chemical, mol% | Mohr's Salt, % | Grafting Chemicals Cone. % | Co γ-ray Radiation, kGy/hr | Raschig ring, Wo, g | DOG, % |
| 183 | PTEE6-4VP/DVB01-IM0.10-50-7KG/15 | 99 | 1 | 0.1 | 50 | 7-15 | 19.5129 | 3.34 |
| 184 | PTEE6-4VP/DVB01-IM0.25-50-7KG/15 | 99 | 1 | 0.25 | 50 | 7-15 | 19.4801 | 3.63 |
| 185 | PTEE6-4VP/DVB01-IM0.50-50-7KG/15 | 99 | 1 | 0.5 | 50 | 7-15 | 19.4288 | 3.77 |
| 186 | PTFE6-4VP/DVB02-IM0.10-50-7KG/15 | 98 | 2 | 0.1 | 50 | 7-15 | 19.5046 | 2.60 |
| 187 | PTFE6-4VP/DVB02-IM0.25-50-7KG/15 | 98 | 2 | 0.25 | 50 | 7-15 | 19.5297 | 2.65 |
| 188 | PTFE6-4VP/DVB02-IM0.50-50-7KG/15 | 98 | 2 | 0.5 | 50 | 7-15 | 19.4700 | 2.62 |
| 189 | PTFE6-4VP/DVB03-IM0.10-50-7KG/15 | 97 | 3 | 0.1 | 50 | 7-15 | 19.4756 | 2.41 |
| 190 | PTFE6-4VP/DVB03-IM0.25-50-7KG/15 | 97 | 3 | 0.25 | 50 | 7-15 | 19.4566 | 2.39 |
| 191 | PTFE6-4VP/DVB03-IM0.50-50-7KG/15 | 97 | 3 | 0.5 | 50 | 7-15 | 19.4919 | 2.53 |
| 192 | PTEE6-4VP/DVB05-IM0.10-50-7KG/15 | 95 | 5 | 0.1 | 50 | 7-15 | 19.5106 | 2.18 |
| 193 | PTEE6-4VP/DVB05-IM0.25-50-7KG/15 | 95 | 5 | 0.25 | 50 | 7-15 | 19.5250 | 2.29 |
| 194 | PTFE6-4VP/DVB05-IM0.50-50-7KG/15 | 95 | 5 | 0.5 | 50 | 7-15 | 19.4488 | 2.13 |
| 195 | PFA6-4VP/DVB01-IM0.10-50-7KG/15 | 99 | 1 | 0.1 | 50 | 7-15 | 23.8094 | 2.52 |
| 196 | PFA6-4VP/DVB01-IM0.25-50-7KG/15 | 99 | 1 | 0.25 | 50 | 7-15 | 23.6750 | 3.14 |

**Table 13**

| | | | | | | | | |
|---|---|---|---|---|---|---|---|---|
| 197 | PFA6-4VP/DVB01-IM0.50-50-7KG/15 | 99 | 1 | 0.5 | 50 | 7-15 | 23.6811 | 3.54 |
| 198 | PFA6-4VP/DVB02-IM0.10-50-7KG/15 | 98 | 2 | 0.1 | 50 | 7-15 | 23.7029 | 1.82 |
| 199 | PFA6-4VP/DVB02-IM0.25-50-7KG/15 | 98 | 2 | 0.25 | 50 | 7-15 | 23.7264 | 1.83 |
| 200 | PFA6-4VP/DVB02-IM0.50-50-7KG/15 | 98 | 2 | 0.5 | 50 | 7-15 | 23.6790 | 1.88 |
| 201 | PFA6-4VP/DVB03-IM0.10-50-7KG/15 | 97 | 3 | 0.1 | 50 | 7-15 | 23.6854 | 1.54 |
| 202 | PFA6-4VP/DVB03-IM0.25-50-7KG/15 | 97 | 3 | 0.25 | 50 | 7-15 | 23.7164 | 1.50 |
| 203 | PFA6-4VP/DVB03-IM0.50-50-7KG/15 | 97 | 3 | 0.5 | 50 | 7-15 | 23.8301 | 1.46 |
| 204 | PFA6-4VP/DVB05-IM0.10-50-7KG/15 | 95 | 5 | 0.1 | 50 | 7-15 | 23.8258 | 1.28 |
| 205 | PFA6-4VP/DVB05-IM0.25-50-7KG/15 | 95 | 5 | 0.25 | 50 | 7-15 | 23.7152 | 1.30 |
| 206 | PFA6-4VP/DVB05-IM0.50-50-7KG/15 | 95 | 5 | 0.5 | 50 | 7-15 | 23.7082 | 1.35 |
| 207 | PTEE6-4VP/DVB01-IM0.10-50-8KG/15 | 99 | 1 | 0.1 | 50 | 8-15 | 19.4592 | 3.42 |
| 208 | PTEE6-4VP/DVB01-IM0.25-50-8KG/15 | 99 | 1 | 0.25 | 50 | 8-15 | 19.5346 | 4.14 |
| 209 | PTEE6-4VP/DVB01-IM0.50-50-8KG/15 | 99 | 1 | 0.5 | 50 | 8-15 | 19.4129 | 4.48 |
| 210 | PTFE6-4VP/DVB02-IM0.10-50-8KG/15 | 98 | 2 | 0.1 | 50 | 8-15 | 19.4078 | 2.70 |
| 211 | PTFE6-4VP/DVB02-IM0.25-50-8KG/15 | 98 | 2 | 0.25 | 50 | 8-15 | 19.4010 | 2.76 |
| 212 | PTFE6-4VP/DVB02-IM0.50-50-8KG/15 | 98 | 2 | 0.5 | 50 | 8-15 | 19.4968 | 2.52 |
| 213 | PTFE6-4VP/DVB03-IM0.10-50-8KG/15 | 97 | 3 | 0.1 | 50 | 8-15 | 19.3950 | 2.28 |

**Table 14**

| | | | | | | | | |
|---|---|---|---|---|---|---|---|---|
| 214 | PTFE6-4VP/DVB03-IM0.25-50-8KG/15 | 97 | 3 | 0.25 | 50 | 8-15 | 19.4528 | 2.29 |
| 215 | PTFE6-4VP/DVB03-IM0.50-50-8KG/15 | 97 | 3 | 0.5 | 50 | 8-15 | 19.4369 | 2.34 |
| 216 | PTFE6-4VP/DVB05-IM0.10-50-8KG/15 | 95 | 5 | 0.1 | 50 | 8-15 | 19.4570 | 2.28 |
| 217 | PTFE6-4VP/DVB05-IM0.25-50-8KG/15 | 95 | 5 | 0.25 | 50 | 8-15 | 19.4083 | 2.15 |
| 218 | PTFE6-4VP/DVB05-IM0.50-50-8KG/15 | 95 | 5 | 0.5 | 50 | 8-15 | 19.3727 | 2.07 |
| 219 | PFA6-4VP/DVB01-IM0.10-50-8KG/15 | 99 | 1 | 0.1 | 50 | 8-15 | 23.6588 | 1.89 |
| 220 | PFA6-4VP/DVB01-IM0.25-50-8KG/15 | 99 | 1 | 0.25 | 50 | 8-15 | 23.7035 | 3.18 |
| 221 | PFA6-4VP/DVB01-IM0.50-50-8KG/15 | 99 | 1 | 0.5 | 50 | 8-15 | 23.7108 | 4.09 |
| 222 | PFA6-4VP/DVB02-IM0.10-50-8KG/15 | 98 | 2 | 0.1 | 50 | 8-15 | 23.6802 | 1.97 |
| 223 | PFA6-4VP/DVB02-IM0.25-50-8KG/15 | 98 | 2 | 0.25 | 50 | 8-15 | 23.6851 | 2.04 |
| 224 | PFA6-4VP/DVB02-IM0.50-50-8KG/15 | 98 | 2 | 0.5 | 50 | 8-15 | 23.6502 | 2.16 |
| 225 | PFA6-4VP/DVB03-IM0.10-50-8KG/15 | 97 | 3 | 0.1 | 50 | 8-15 | 23.6769 | 2.72 |
| 226 | PFA6-4VP/DVB03-IM0.25-50-8KG/15 | 97 | 3 | 0.25 | 50 | 8-15 | 23.8134 | 1.64 |
| 227 | PFA6-4VP/DVB03-IM0.50-50-8KG/15 | 97 | 3 | 0.5 | 50 | 8-15 | 23.6793 | 0.74 |
| 228 | PFA6-4VP/DVB05-IM0.10-50-8KG/15 | 95 | 5 | 0.1 | 50 | 8-15 | 23.7100 | 2.38 |
| 229 | PFA6-4VP/DVB05-IM0.25-50-8KG/15 | 95 | 5 | 0.25 | 50 | 8-15 | 23.7068 | 1.42 |
| 230 | PFA6-4VP/DVB05-IM0.50-50-8KG/15 | 95 | 5 | 0.5 | 50 | 8-15 | 23.7140 | 1.44 |

**Table 15**

| | | | | | | | | |
|---|---|---|---|---|---|---|---|---|
| 231 | PTFE6-4VP/DVB01-IM0.10-50-9KG/15 | 99 | 1 | 0.1 | 50 | 9-15 | 19.4890 | 3.19 |
| 232 | PTFE6-4VP/DVB01-IM0.25-50-9KG/15 | 99 | 1 | 0.25 | 50 | 9-15 | 19.4267 | 4.16 |
| 233 | PTFE6-4VP/DVB01-IM0.50-50-9KG/15 | 99 | 1 | 0.5 | 50 | 9-15 | 19.4690 | 3.86 |
| 234 | PTFE6-4VP/DVB05-IM0.10-50-9KG/15 | 95 | 5 | 0.1 | 50 | 9-15 | 19.4064 | 2.59 |
| 235 | PTFE6-4VP/DVB05-IM0.25-50-9KG/15 | 95 | 5 | 0.25 | 50 | 9-15 | 19.4450 | 2.72 |
| 236 | PTFE6-4VP/DVB05-IM0.50-50-9KG/15 | 95 | 5 | 0.5 | 50 | 9-15 | 19.5700 | 2.82 |
| 243 | PFA6-4VP/DVB01-IM0.10-50-9KG/15 | 99 | 1 | 0.1 | 50 | 9-15 | 23.7344 | 3.25 |
| 244 | PFA6-4VP/DVB01-IM0.25-50-9KG/15 | 99 | 1 | 0.25 | 50 | 9-15 | 23.6704 | 2.34 |
| 245 | PFA6-4VP/DVB01-IM0.50-50-9KG/15 | 99 | 1 | 0.5 | 50 | 9-15 | 23.7042 | 2.72 |
| 246 | PFA6-4VP/DVB05-IM0.10-50-9KG/15 | 95 | 5 | 0.1 | 50 | 9-15 | 23.7290 | 1.94 |
| 247 | PFA6-4VP/DVB05-IM0.25-50-9KG/15 | 95 | 5 | 0.25 | 50 | 9-15 | 23.7007 | 1.93 |
| 248 | PFA6-4VP/DVB05-IM0.50-50-9KG/15 | 95 | 5 | 0.5 | 50 | 9-15 | 23.6755 | 2.01 |

### Example 6

PTFE10-4VPIM0.5-40-5KG/15 (DOG%; 3.3) and PTFE10-4VPIM1.0-60-5KG/15 (DOG%; 2.17), which are the 4-VP-grafted Teflon support samples prepared in Example 1, were used as rhodium complex ion catalyst supports for methanol carbonylation. A high-pressure rector system for acetic acid synthesis was configured such that carbon monoxide adjusted to 40 bar was fed upward from a CO cylinder through a 1/8" 316SS line via a check valve into a 160-cc high-pressure Teflon reactor (50 mm diameter and 80 mm depth) equipped with a magnetic stirrer and a temperature controller. In addition, in order to introduce reactants, the upper portion of the reactor system included a 1/4" ball valve, a plug and a thermocouple thermometer. In addition, the reactor system was equipped with a pressure indicator, a temperature control indicator and an RPM indicator showing stirring speed. In order to initiate a reaction, a magnetic bar (35 mm length and 11 mm diameter) was first placed in the reactor, and a cylindrical titanium mesh cage (about 18 mesh size) was disposed in contact with the inner wall of the reactor. Then, a rhodium catalyst (RhCl₃) was placed in the reactor in an amount of about 73 mg corresponding to 800 mg based on about 45 g of a reactant composition, and a circular titanium mesh (50 mm diameter and 8 mesh size) having 2 mm x 4 mm rhombus holes formed therein was covered thereon, after which the Raschig ring sample which is the Teflon catalyst support grafted with 4-VP was placed evenly thereon. The reactor was capped and subjected to an air tightness test by filling nitrogen to a pressure of 40 bar, after which the internal gas of the reactor was completely removed under vacuum, and then the reactor was adjusted to about 1.2 bar by carbon monoxide. A reactant composition comprising 32.5 wt% of methanol, 56.4 wt% of acetic acid, 6.5 wt% of methyl iodide and 4.6 wt% of water was prepared, and 45 g of the reactant composition was taken by a syringe. The ball valve plug in the upper portion of the reactor was opened, and the valve was opened slowly such that carbon monoxide was vented out slowly. Then, the valve was completely opened, and the reactant in the syringe was rapidly introduced into the reactor. Next, the ball valve was closed and completely sealed with the plug, and stirring at 1250 rpm was started. The temperature of the reactor was elevated to 190°C, and when the temperature was stabilized, the valve connected to the reactor was opened and carbon monoxide adjusted to 40 bar was introduced into the reactor to initiate the reaction. After 3 hours of the reaction, a carbon monoxide gas line was blocked, and the reactor was separated from a heating mantle and cooled rapidly with cold water, and the weight of the product was measured. The reactor was disassembled, and the product was analyzed by GC. Furthermore, the Raschig ring rhodium complex ion support catalyst was recovered, washed with methanol, dried completely in a vacuum dryer at 80°C, and reused as a rhodium complex ion catalyst in the next reaction. The first reaction performed as described above was defined as a zero-order reaction, and the PTFE Raschig ring rhodium complex ion catalyst obtained therein was used as a catalyst in the next first-order reaction. In the next reaction, among the reaction preparation procedures as described above, the rhodium salt was not introduced, and the PTFE Raschig ring rhodium complex ion catalyst obtained in the zero-order reaction was introduced, and a predetermined amount of the reactant composition having the same composition as described above was introduced according to a predetermined order, after which the reaction was performed and the weight of the product was measured and analyzed. The results were defined as first-order reaction results, and the catalyst used was recovered, washed with methanol, dried, and then used in a second-order reaction. Table 16 shows the results of measuring the conversion rate of methanol and the yields of acetic acid, methyl acetate and water in the products obtained by the acetic acid synthesis reaction performed using the 10 mm PTFE Raschig ring rhodium complex ion catalyst support. In Example 6, the liquid stirred by the magnetic bar came into contact with carbon monoxide via the Raschig ring placed on the mesh, and thus mass transfer was not smooth and the reaction rate was very slower than the reaction rate of a homogeneous catalyst. Accordingly, these results were used to compare relative results according to the DOG% of the same catalyst support. It can be seen that when DOG% was 2% or more, the conversion rate of methanol was 99% or more and the yield of acetic acid was 60% or more. In addition, it can be seen that when the reaction time in a third-order experiment was extended to 6 hours, the yield of acetic acid increased to 90% or more, and as the reaction order increased, the loss of rhodium decreased to about 1%. The results are summarized in Table 16 below.

**Table 16**

| Example 6 | | | | | | | | | | |
|---|---|---|---|---|---|---|---|---|---|---|
| Experimental order | Catalyst | Rh | Reaction conditions | Reactant | Product | Conversion % | Yield % | | | Rh Loss % |
| | No/g/ER/DOG%/RhCl₃ (mg) | ppm | °C/bar/hr | g | g | MeOH | AcOH | AcOMe | H₂O | |
| 1 | PTFE10-4VPIM0.5-40-5KG/15(16/17.4447/ 28/3.33/73.0) | 800 | 190/40/3 | 45.3929 | 54.3714 | 99.58 | 65.90 | 15.75 | 28.51 | 3.81 |
| 2 | | 800 | 190/40/3 | 45.1402 | 54.4119 | 99.29 | 63.61 | 18.16 | 32.84 | 3.73 |
| 3 | | 800 | 190/40/6 | 45.2577 | 56.3698 | 99.37 | 90.64 | 3.05 | 1.58 | 1.03 |
| 1 | PTFE10-4VPIM1 .0-60-5KG/15(20/17.4324/ 28/2.17/73.1) | 800 | 190/40/3 | 45.0310 | 53.7438 | 99.59 | 63.75 | 16.86 | 26.89 | 3.81 |
| 2 | | 800 | 190/40/3 | 45.1514 | 55.8185 | 99.24 | 62.30 | 19.59 | 35.06 | 4.07 |
| 3 | | 800 | 190/40/5.5 | 44.9921 | 56.0549 | 99.20 | 89.17 | 4.04 | 3.41 | 1.58 |

### Example 7

The following 4-VP-grafted PTFE catalyst support samples prepared in Example 2 were used as rhodium complex ion catalyst supports for methanol carbonylation: PTFE6-4VPIM0.10-50-5KG/15 (DOG%; 4.99); PTFE6-4VPIM0.25-50-5KG/15 (DOG%; 5.82); PTFE6-4VPIM0.10-50-6KG/15 (DOG%; 5.66); PTFE6-4VPIM0.10-50-6KG/15 (DOG%; 5.93); PTFE6-4VPIM0.10-50-7KG/15 (DOG%; 5.79); and PTFE6-4VPIM0.25-50-7KG/15 (DOG%; 6.0). As described in Example 6, a high-pressure reactor system for acetic acid synthesis was used. The experiment was prepared in the same manner as described in Example 6, except that the rhodium catalyst (RhCl₃) was introduced in amounts of about 73.0 mg and 36.2 mg corresponding to 800 ppm and 400 ppm, respectively, based on about 45 g of the reactant composition, and that a circular titanium mesh (50 mm diameter and 8 mesh size) having 2 mm x 4 mm rhombus holes formed therein was covered thereon, after which a selected Raschig ring sample which is the Teflon catalyst support grafted with 4-VP was placed evenly thereon. The reactor was capped and subjected to an air tightness test by filling nitrogen to a pressure of 40 bar, after which the internal gas of the reactor was completely removed under vacuum, and then the reactor was adjusted to about 1.2 bar by carbon monoxide. A reactant composition comprising 32.5 wt% of methanol, 56.4 wt% of acetic acid, 6.5 wt% of methyl iodide and 4.6 wt% of water was prepared, and 45 g of the reactant composition was taken by a syringe. The ball valve plug in the upper portion of the reactor was opened, and the valve was opened slowly such that carbon monoxide was vented out slowly. Then, the valve was completely opened, and the reactant in the syringe was rapidly introduced into the reactor. Next, the ball valve was closed and completely sealed with the plug, and stirring at 1250 rpm was started. The internal temperature of the reactor was elevated to 190°C, and when the temperature was stabilized, the valve connected to the reactor was opened and carbon monoxide adjusted to 40 bar was introduced into the reactor to initiate the reaction. After 3 hours of the reaction, a carbon monoxide gas line was blocked, and the reactor was separated from a heating mantle and cooled rapidly with cold water, and the weight of the product was measured. The reactor was disassembled, and the product was analyzed by GC. Furthermore, the 6 mm PTFE Raschig ring rhodium complex ion catalyst was recovered, washed with methanol, dried completely in a vacuum dryer at 80°C, and reused as a catalyst in the next reaction. The first reaction performed as described above was defined as a zero-order reaction, and the 6 mm PTFE Raschig ring rhodium complex ion catalyst obtained therein was used as a catalyst in the next first-order reaction. In the next reaction, among the reaction preparation procedures as described above, the rhodium salt was not introduced, and the 6 mm PTFE Raschig ring rhodium complex ion catalyst obtained in the zero-order reaction was introduced, and a predetermined amount of the reactant composition having the same composition as described above was introduced according to a predetermined order, after which the reaction was performed and the weight of the product was measured and analyzed. The results were defined as first-order reaction results, and the catalyst used was recovered, washed with methanol, dried, and then used in a second-order reaction. Table 17 below shows the results of measuring the conversion rate of methanol and the yields of acetic acid, methyl acetate and water in the products obtained by the acetic acid synthesis reaction performed using the 6 mm PTFE Raschig ring catalyst support. In Example 7, the liquid stirred by the magnetic bar came into contact with carbon monoxide via the Raschig ring placed on the mesh, and thus mass transfer was not smooth and the reaction rate was very slower than the reaction rate of a homogeneous catalyst. Accordingly, these results were used to compare relative results according to the DOG% of the same catalyst support. It can be seen that when DOG% was about 6%, the conversion rate of methanol 99% or more, the yield of acetic acid in the first-order reaction was 40% or more, and the yield of acetic acid in the second-order reaction increased to about 60% or more. In addition, it can be seen that the loss of the rhodium ion decreased to 1% or less.

**Table 17**

| Example 7 | | | | | | | | | | |
|---|---|---|---|---|---|---|---|---|---|---|
| Order | Catalyst | Rh | Reaction conditions | Reactant | Product | Conversion % | Yield % | | | Rh Loss % |
| | No/g/EA/DOG%/ RhCl₃(mg) | ppm | °C/bar/hr | G | g | MeOH | AcOH | AcOMe | H₂O | |
| 1 | PTFE6-4VPIM0.10-50-KG/15(63/20.3722/ 100/4.99/63.5) | 400 | 190/40/3 | 45.212 | 53.196 | 98.32 | 47.06 | 22.28 | 29.30 | 3.25 |
| 1 | PTFE6-4VPIM0.25-50-5KG/15(64/20.4593/ 100/5.82/73.4) | 800 | 190/40/3 | 45.128 | 51.483 | 97.81 | 38.13 | 24.14 | 31.34 | 5.77 |
| 2 | | 800 | 190/40/3 | 45.649 | 54.381 | 99.13 | 45.50 | 20.22 | 18.47 | 1.41 |
| 1 | PTFE6-4VPIM0.10-50-6KG/15(79/20.4538/ 100/5.66/36.7) | 400 | 190/40/3 | 45.502 | 52.824 | 97.78 | 39.34 | 25.48 | 31.77 | 3.61 |
| 2 | | 400 | 190/40/3 | 44.968 | 53.952 | 98.92 | 57.98 | 17.54 | 18.84 | 0.24 |
| 1 | PTFE6-4VPIM0.10-50-6KG/15(80/20.5010/ 100/5.93/73.4) | 800 | 190/40/3 | 44.861 | 53.555 | 99.28 | 62.12 | 14.88 | 18.35 | 1.98 |
| 1 | PTFE6-4VPIM0.10-50-7KG/15(95/20.4891/ 100/5.79/36.8) | 400 | 190/40/3 | 46.087 | 54.311 | 99.02 | 50.85 | 50.85 | 20.08 | 3.23 |
| 2 | | 400 | 190/40/5 | 45.616 | 56.064 | 99.64 | 77.61 | 77.61 | 9.10 | 0.20 |
| 1 | PTEFE6-4VPIM0.25-50-7KG/15(96/20.5998/ 100/6.00/73.1) | 800 | 190/40/3 | 45.310 | 51.785 | 97.60 | 36.72 | 25.24 | 32.86 | 4.91 |

### Example 8

The following 4-VP-grafted PTFE catalyst support samples prepared in Example 5 were used as rhodium complex ion catalyst supports for methanol carbonylation: PTFE6-4VP/DVB01-IM0.10-50-7KG/15 (DOG%; 3.34); PTFE6-4VP/DVB01-IM0.25-50-7KG/15 (DOG%; 3.63); PTFE6-4VP/DVB01-IM0.50-50-7KG/15 (DOG%; 3.77); PTFE6-4VP/DVB05-IM0.10-50-7KG/15 (DOG%; 2.18); PTFE6-4VP/DVB05-IM0.50-50-7KG/15 (DOG%; 2.13); PTFE6-4VP/DVB01-IM0.10-50-8KG/15 (DOG%; 3.42); PTFE6-4VP/DVB01-IM0.25-50-8KG/15 (DOG%; 4.14); PTFE6-4VP/DVB01-IM0.50-50-8KG/15 (DOG%; 4.48); PTFE6-4VP/DVB05-IM0.10-50-8KG/15 (DOG%; 2.28); PTFE6-4VP/DVB05-IM0.50-50-8KG/15 (DOG%; 2.07); PTFE6-4VP/DVB01-IM0.01-50-9KG/15 (DOG%; 3.19); PTFE6-4VP/DVB01-IM0.25-50-9KG/15(DOG%; 4.16); and PTFE6-4VP/DVB01-IM0.50-50-9KG/15(DOG%; 3.86). As described in Example 6, a high-pressure reactor system for acetic acid synthesis was used. The experiment was prepared in the same manner as described in Example 6, except that the rhodium catalyst (RhCl₃) was introduced in an amount of about 36.2 mg corresponding to 400 ppm based on about 45 g of a reactant composition, and that a circular titanium mesh (50 mm diameter and 8 mesh size) having 2 mm x 4 mm rhombus holes formed therein was covered thereon, after which a selected 6 mm PTEF Raschig ring catalyst support grafted with 4-VP/DVB was placed evenly thereon. The reactor was capped and subjected to an air tightness test by filling nitrogen to a pressure of 40 bar, after which the internal gas of the reactor was completely removed under vacuum, and then the reactor was adjusted to about 1.2 bar by carbon monoxide. A reactant composition comprising 32.5 wt% of methanol, 56.4 wt% of acetic acid, 6.5 wt% of methyl iodide and 4.6 wt% of water was prepared, and 45 g of the reactant composition was taken by a syringe. The ball valve plug in the upper portion of the reactor was opened, and the valve was opened slowly such that carbon monoxide was vented out slowly. Then, the valve was completely opened, and the reactant in the syringe was rapidly introduced into the reactor. Next, the ball valve was closed and completely sealed with the plug, and stirring at 1250 rpm was started. The internal temperature of the reactor was elevated to 190°C, and when the temperature was stabilized, the valve connected to the reactor was opened and carbon monoxide adjusted to 40 bar was introduced into the reactor to initiate the reaction. After the reaction, a carbon monoxide gas line was blocked, and the reactor was separated from a heating mantle and cooled rapidly with cold water, and the weight of the product was measured. The reactor was disassembled, and the product was analyzed by GC. Furthermore, the 6 mm PTFE Raschig ring rhodium complex ion catalyst was recovered, washed with methanol, dried completely in a vacuum dryer at 80°C, and reused as a catalyst in the next reaction. The first reaction performed as described above was defined as a zero-order reaction, and the 6 mm PTFE Raschig ring rhodium complex ion catalyst obtained therein was used as a catalyst in the next first-order reaction. In the next reaction, among the reaction preparation procedures as described above, the rhodium salt was not introduced, and the 6 mm PTFE Raschig ring rhodium complex ion catalyst obtained in the zero-order reaction was introduced, and a predetermined amount of the reactant composition having the same composition as described above was introduced according to a predetermined order, after which the reaction was performed and the weight of the product was measured and analyzed. The results were defined as first-order reaction results, and the catalyst used was recovered, washed with methanol, dried, and then used in a second-order reaction. Tables 18 and 19 below show the results of measuring the conversion rate of methanol and the yields of acetic acid, methyl acetate and water in the products obtained by the acetic acid synthesis reaction performed using the 6 mm PTFE Raschig ring rhodium complex ion catalyst. These results are merely used to compare relative results according to the DOG % of the same catalyst support, because it is obvious that the liquid stirred by the magnetic bar comes into contact with carbon monoxide via the Raschig ring placed on the mesh, and thus mass transfer is not smooth and the reaction rate is very slower than the reaction rate of a homogeneous catalyst. It can be seen that when DOG% was about 3% to 4%, the conversion rate of methanol was 99% or more, the yield of acetic acid in the first-order reaction was 80% or more, and the yield of acetic acid in the second to tenth-order reactions was also maintained at about 80%. In addition, it can be seen that the loss of the rhodium ion was about 1% or less even in about 10 reactions. The above experimental results indicate that the PTFE catalyst support obtained by irradiating cobalt gamma rays to the grafting solution prepared by diluting the crosslinking agent divinylbenzene in an amount of 1 mol% to 5 mol% based on vinylpyridine shows better results. The results are shown in Tables 18 and 19 below.

**Table 18**

| Example 8 | | | | | | | | | | |
|---|---|---|---|---|---|---|---|---|---|---|
| Order | Catalyst | Rh | Reaction conditions | Reactant | Product | Convrsion % | Yield % | | | Rh Loss % |
| | No/g/ER/DOGt/KhCl₃ (mg) | ppm | °C/bar/hr | g | g | MeOH | AcOH | AcOMe | H₂O | |
| 1 | PTFE6-4VP/DJB01-IM0.10-50-7KG/15 (183/20.165/100/3. 34/36.8) | 400 | 190/40/3 | 45.297 | 55.491 | 99.61 | 80.77 | 7.17 | 5.31 | 0.35 |
| 1 | PTFE6-4VP/DVB01-IM0.25-50-KG/15 (184/20.188/100/3. 63/36.9) | 400 | 190/40/3 | 45.297 | 55.593 | 99.67 | 81.97 | 6.76 | 5.89 | 0.59 |
| 1 | PTFE6-4VP/DVB01-IM0.50-50-7KG/15 (185/20.161/100/3. 77/36.7) | 400 | 190/40/3 | 45.254 | 55.602 | 99.70 | 80.77 | 7.50 | 6.19 | 0.76 |
| 1 | PTFE6-4VP/DVB01-IM0.10-50-8KG/15 ( 207/20.124/100/3.4 2/37.1) | 400 | 190/40/3 | 45.337 | 55.823 | 99.68 | 81.56 | 7.43 | 5.03 | 0.44 |
| 1 | PTFE6-4VP/DVB01-IM0.25-50-8KG/15 (208/20.344/100/4. 14/36.8) | 400 | 190/40/3 | 45.066 | 55.445 | 99.74 | 85.00 | 5.48 | 4.12 | 0.49 |
| 2 | | 400 | 190/40/3 | 45.052 | 55.247 | 99.85 | 79.47 | 7.77 | 7.42 | 0.29 |
| 3 | | 400 | 190/40/3 | 45.269 | 55.462 | 99.63 | 81.80 | 6.31 | 4.99 | N.D |
| 4 | | 400 | 190/40/3 | 45.037 | 55.482 | 99.76 | 82.47 | 6.60 | 6.84 | 0.24 |
| 5 | | 400 | 190/40/3 | 45.288 | 55.746 | 99.77 | 81.81 | 6.89 | 6.14 | N.D |
| 6 | | 400 | 190/40/3 | 45.136 | 55.278 | 99.78 | 81.91 | 6.51 | 6.31 | N.D |
| 7 | | 400 | 190/40/3 | 45.093 | 54.997 | 99.82 | 79.19 | 7.44 | 7.20 | N.D |
| 8 | | 400 | 190/40/3 | 44.999 | 54.980 | 99.79 | 79.62 | 7.57 | 7.03 | N.D |
| 9 | | 400 | 190/40/3 | 45.179 | 55.229 | 99.46 | 81.15 | 7.11 | 7.58 | 0.30 |
| 10 | | 400 | 190/40/3 | 45.410 | 55.341 | 99.41 | 78.84 | 7.74 | 7.40 | N.D |

**Table 19**

| | | | | | | | | | | |
|---|---|---|---|---|---|---|---|---|---|---|
| 1 | PTFE6-4VP/DVB01-IM0.50-50-8KG/15 (209/20.283/100/ 4.48/36.7) | 400 | 190/40/3 | 45.630 | 55.727 | 99.59 | 75.94 | 9.65 | 10.36 | 6.47 |
| 1 | PTFE6-4VP/DVB01-IM0.10-50-9KG/15 (231/20.111/100/ 3.19/37.0) | 400 | 190/40/3 | 45.368 | 55.949 | 99.65 | 83.42 | 6.44 | 5.31 | 0.58 |
| 1 | PTFE6-4VP/DVB01-IM0.25-50-9KG/15 (232/20.235/100/4.16/36.9) | 400 | 190/40/3 | 45.053 | 55.847 | 99.72 | 88.75 | 4.19 | 2.18 | 0.42 |
| 2 | | 400 | 190/40/3 | 45.373 | 56.070 | 99.65 | 82.60 | 6.27 | 4.43 | 0.29 |
| 3 | | 400 | 190/40/3 | 45.203 | 55.515 | 99.72 | 82.28 | 6.16 | 5.07 | 0.20 |
| 4 | | 400 | 190/40/3 | 44.961 | 55.492 | 99.77 | 83.06 | 6.41 | 6.10 | 0.56 |
| 5 | | 400 | 190/40/3 | 45.326 | 55.940 | 99.79 | 84.46 | 6.15 | 6.03 | 0.02 |
| 6 | | 400 | 190/40/3 | 45.039 | 55.195 | 99.85 | 81.10 | 6.76 | 6.24 | 0.20 |
| 7 | | 400 | 190/40/3 | 45.077 | 55.120 | 99.81 | 80.89 | 7.09 | 6.89 | 0.20 |
| 8 | | 400 | 190/40/3 | 45.285 | 55.347 | 99.80 | 81.20 | 6.59 | 5.62 | 0.08 |
| 9 | | 400 | 190/40/3 | 45.216 | 55.305 | 99.74 | 78.86 | 7.49 | 6.11 | 0.40 |
| 10 | | 400 | 190/40/3 | 45.208 | 55.154 | 99.82 | 78.64 | 7.80 | 7.31 | 0.15 |
| 1 | PTFE6-4VP/DVB01-IM0.50-50-9KG/15 (233/20.221/100/3.86/36.6) | 400 | 190/40/3 | 45.165 | 55.967 | 99.70 | 83.91 | 6.07 | 7.72 | 6.47 |

### Example 9

The following 4-VP-grafted PTFE catalyst support samples prepared in Example 5 were used as rhodium complex ion catalyst supports for methanol carbonylation: PFA6-4VP/DVB01-IM0.10-50-7KG/15 (DOG%; 2.52); PFA6-4VP/DVB01-IM0.25-50-7KG/15 (DOG%; 3.14); PFA6-4VP/DVB01-IM0.50-50-7KG/15 (DOG%; 3.54); PFA6-4VP/DVB05-IM0.10-50-7KG/15 (DOG%; 1.28); PFA6-4VP/DVB05-IM0.50-50-7KG/15 (DOG%; 1.35); PFA6-4VP/DVB01-IM0.10-50-8KG/15 (DOG%; 1.89); PFA6-4VP/DVB01-IM0.25-50-8KG/15 (DOG%; 3.18); PFA6-4VP/DVB01-IM0.50-50-8KG/15 (DOG%; 4.09); PFA6-4VP/DVB01-IM0.10-50-9KG/15 (DOG%; 3.25); PFA6-4VP/DVB01-IM0.25-50-9KG/15 (DOG%; 2.34); PFA6-4VP/DVB01-IM0.50-50-9KG/15 (DOG; 2.72); PFA6-4VP/DVB02-IM0.10-50-9KG/15 (DOG; 1.94); PFA6-4VP/DVB02-IM0.25-50-9KG/15 (DOG%; 1.93); PFA6-4VP/DVB03-IM0.10-50-9KG/15 (DOG%; 1.56); PFA6-4VP/DVB03-IM0.25-50-9KG/15 (DOG%; 1.71); PFA6-4VP/DVB05-IM0.10-50-9KG/15 (DOG%; 1.38); and PFA6-4VP/DVB05-IM0.25-50-9KG/15 (DOG%; 1.40). As described in Example 6, a high-pressure reactor system for acetic acid synthesis was used. The experiment was prepared in the same manner as described in Example 6, except that the rhodium catalyst (RhCl₃) was introduced in an amount of about 36.7 mg corresponding to 400 ppm based on about 45 g of a reactant composition, and that a circular titanium mesh (50 mm diameter and 8 mesh size) having 2 mm x 4 mm rhombus holes formed therein was covered thereon, after which a Raschig ring which is a selected PFA Raschig catalyst support grafted with 4-VP/DVB was placed evenly thereon. The reactor was capped and subjected to an air tightness test by filling nitrogen to a pressure of 40 bar, after which the internal gas of the reactor was completely removed under vacuum, and then the reactor was adjusted to about 1.2 bar by carbon monoxide. A reactant composition comprising 32.5 wt% of methanol, 56.4 wt% of acetic acid, 6.5 wt% of methyl iodide and 4.6 wt% of water was prepared, and 45 g of the reactant composition was taken by a syringe. The ball valve plug in the upper portion of the reactor was opened, and the valve was opened slowly such that carbon monoxide was vented out slowly. Then, the valve was completely opened, and the reactant in the syringe was rapidly introduced into the reactor. Next, the ball valve was closed and completely sealed with the plug, and stirring at 1250 rpm was started. The internal temperature of the reactor was elevated to 190°C, and when the temperature was stabilized, the valve connected to the reactor was opened and carbon monoxide adjusted to 40 bar was introduced into the reactor to initiate the reaction. After the reaction, a carbon monoxide gas line was blocked, and the reactor was separated from a heating mantle and cooled rapidly with cold water, and the weight of the product was measured. The reactor was disassembled, and the product was analyzed by GC. Furthermore, the 6 mm PFA Raschig ring rhodium complex ion catalyst was recovered, washed with methanol, dried completely in a vacuum dryer at 80°C, and reused as a catalyst in the next reaction. The first reaction performed as described above was defined as a zero-order reaction, and the 6 mm PFA Raschig ring rhodium complex ion catalyst obtained therein was used as a catalyst in the next first-order reaction. In the next reaction, among the reaction preparation procedures as described above, the rhodium salt was not introduced, and the 6 mm PFA Raschig ring rhodium complex ion catalyst obtained in the zero-order reaction was introduced, and a predetermined amount of the reactant composition having the same composition as described above was introduced according to a predetermined order, after which the reaction was performed and the weight of the product was measured and analyzed. The results were defined as first-order reaction results, and the catalyst used was recovered, washed with methanol, dried, and then used in a second-order reaction. Tables 20 and 21 below show the results of measuring the conversion rate of methanol and the yields of acetic acid, methyl acetate and water in the products obtained by the acetic acid synthesis reaction performed using the 6 mm PFA Raschig ring rhodium complex ion catalyst. These results are merely used to compare relative results according to the DOG % of the same catalyst support, because it is obvious that the liquid stirred by the magnetic bar comes into contact with carbon monoxide via the Raschig ring placed on the mesh, and thus mass transfer is not smooth and the reaction rate is very slower than the reaction rate of a homogeneous catalyst. It can be seen that when DOG% was about 1% to 4%, the conversion rate of methanol was 99% or more, the yield of acetic acid in the first-order reaction was 80% or more, the yield of acetic acid in the second to tenth-order reactions was maintained at about 80%, and the loss of the rhodium ion was about 1% or less. The above experimental results indicate that the PFA support catalyst obtained by irradiating cobalt gamma rays to the grafting solution prepared by diluting the crosslinking agent divinylbenzene in an amount of 1 mol% to 5 mol% based on vinylpyridine shows better results. The results are shown in Tables 20 and 21 below.

**Table 20**

| Example 9 | | | | | | | | | | |
|---|---|---|---|---|---|---|---|---|---|---|
| Expement-1 order | Catalyst | Rh | Reaction conditions | Reactant | Product | Conversion % | Yield % | | | Rh Loss % |
| | No/g/EA/DOG%/RhCl₃ (mg) | ppm | °C/bar/hr | g | g | MeOH | AcOH | AcCMe | H₂O | |
| 1 | PFA6-4VP/DVB01-IM0.10-50-7KG/15(195/24.285/ 100/2.52/36.9) | 400 | 190/40/3 | 44.998 | 55.108 | 99.72 | 80.13 | 7.57 | 6.95 | 1.02 |
| 1 | PFA6-4VP/DVB01-IM0.25-50-7KG/15(196/24.303/ 100/3.14/36.8) | 400 | 190/40/3 | 45.527 | 55.951 | 99.62 | 79.89 | 7.86 | 7.20 | 0.91 |
| 1 | PFA6-4VP/DVB01-IM0.50-50-7KG/15(197/24.336/ 100/3.54/36.6) | 400 | 190/40/3 | 45.512 | 56.708 | 99.64 | 90.46 | 3.39 | 0.90 | 0.53 |
| 2 | | 400 | 190/40/3 | 45.432 | 54.829 | 99.80 | 77.95 | 7.26 | 6.26 | 0.16 |
| 3 | | 400 | 190/40/3 | 45.065 | 55.571 | 99.75 | 83.46 | 6.35 | 5.13 | 0.09 |
| 4 | | 400 | 190/40/3 | 45.203 | 55.144 | 99.79 | 79.87 | 7.07 | 6.80 | 0.18 |
| 5 | | 400 | 190/40/3 | 45.310 | 55.194 | 99.83 | 78.56 | 7.60 | 7.19 | 0.38 |
| 6 | | 400 | 190/40/3 | 45.370 | 55.041 | 99.85 | 73.86 | 9.81 | 10.82 | 0.06 |
| 7 | | 400 | 190/40/3 | 45.336 | 55.126 | 99.74 | 74.87 | 9.98 | 10.78 | 0.10 |
| 8 | | 400 | 190/40/3 | 45.554 | 53.676 | 99.80 | 66.86 | 11.12 | 11.10 | 0.14 |
| 9 | | 400 | 190/40/3 | 45.576 | 54.717 | 99.34 | 67.16 | 12.67 | 14.90 | 0.33 |
| 10 | | 400 | 190/40/3 | 45.440 | 52.692 | 99.29 | 55.91 | 15.79 | 19.27 | 1.02 |
| 1 | PFA6-4VP/DVB05-IM0.10-50-7KG/15(204/24.132/ 100/1.28/37 . 2) | 400 | 190/40/3 | 45.385 | 54.610 | 99.77 | 69.96 | 13.50 | 21.56 | 2.22 |
| 1 | PFA6-4VP/DVB05-IM0.50-50-7KG/15(206/24.028/ 100/1.35/36.9) | 400 | 190/40/3 | 45.209 | 54.818 | 99.52 | 71.25 | 13.57 | 23.68 | 1.53 |
| 1 | PFA6-4VP/DVB01-IM0.10-50-8KG/15(219/24.106/ 100/1.89/36.7) | 400 | 190/40/3 | 45.140 | 55.296 | 99.70 | 81.97 | 6.94 | 12.37 | 0.81 |

**Table 21**

| | | | | | | | | | | |
|---|---|---|---|---|---|---|---|---|---|---|
| 1 | PFA6-4VP/DVB01-IM0.25-50-8KG/15(220/24.457/ 100/3.18/36.7) | 400 | 190/40/3 | 45.173 | 55.576 | 99.72 | 82.39 | 6.87 | 6.51 | 1.01 |
| 1 | PFA6-4VP/DVB01-IM0.50-50-8KG/15(221/24.680/ 100/4.09/37.1) | 400 | 190/40/3 | 45.697 | 57.236 | 99.70 | 93.38 | 2.05 | 0.00 | 0.59 |
| 2 | | 400 | 190/40/3 | 44.993 | 55.461 | 99.85 | 85.22 | 5.03 | 3.76 | 0.07 |
| 3 | | 400 | 190/40/3 | 45.430 | 55.650 | 99.79 | 83.87 | 5.19 | 3.50 | 0.10 |
| 4 | | 400 | 190/40/3 | 45.263 | 55.814 | 99.76 | 86.27 | 4.51 | 3.05 | 0.11 |
| 5 | | 400 | 190/40/3 | 45.712 | 54.847 | 99.86 | 76.26 | 7.50 | 6.89 | 0.34 |
| 6 | | 400 | 190/40/3 | 45.105 | 54.847 | 99.89 | 80.51 | 6.75 | 6.04 | 0.00 |
| 7 | | 400 | 190/40/3 | 45.064 | 54.255 | 99.89 | 76.35 | 8.11 | 7.30 | 0.00 |
| 8 | | 400 | 190/40/3 | 45.148 | 55.341 | 99.75 | 78.47 | 8.54 | 8.54 | 0.00 |
| 9 | | 400 | 190/40/3 | 45.022 | 54.596 | 99.83 | 72.68 | 10.5 0 | 11.64 | 0.04 |
| 10 | | 400 | 190/40/3 | 45.163 | 54.576 | 99.47 | 69.47 | 12.0 3 | 13.93 | 0.36 |
| 1 | PFA6-4VP/DVB01-IM0.10-50-9KG/15(243/24.505/ 100/3.25/36.5) | 400 | 190/40/3 | 45.875 | 56.454 | 99.77 | 82.21 | 6.87 | 6.57 | 0.83 |
| 1 | PFA6-4VP/DVB01-IMO.25-50-9KG/15(244/24.223/ 100/2.34/36.9) | 400 | 190/40/3 | 45.142 | 55.649 | 99.79 | 83.34 | 6.44 | 5.78 | 0.81 |
| 1 | PFA6-4VP/DVB01-IM0.50-50-9KG/15(245/24.348/ 100/2.72/37.0) | 400 | 190/40/3 | 45.081 | 55.154 | 99.78 | 80.63 | 6.81 | 7.99 | 1.43 |

### Example 10

The following 4-VP/acrylic acid-grafted PTFE catalyst support samples prepared in Example 4 were used as rhodium complex ion supports for methanol carbonylation: PTFE6-4VP/AcAc35-IM0.25-50-5KG/15 (DOG%; 0.13); PTFE6-4VP/AcAc10-IM0.50-50-5KG/15 (DOG%; 2.51); PTFE6-4VP/AcAc10-IM0.10-50-6KG/15 (DOG%; 3.66); and PTFE6-4VP/AcAc10-IM0.10-50-7KG/15(DOG%; 4.52). As described in Example 6, a high-pressure reactor system for acetic acid synthesis was used. The experiment was prepared in the same manner as described in Example 6, except that the rhodium catalyst (RhCl₃) was introduced in an amount of about 36.7 mg corresponding to 400 ppm based on about 45 g of a reactant composition, and that a circular titanium mesh (50 mm diameter and 8 mesh size) having 2 mm x 4 mm rhombus holes formed therein was covered thereon, after which a selected 6 mm PTEF Raschig ring catalyst support grafted with 4-VP/AcAc was placed evenly thereon. The reactor was capped and subjected to an air tightness test by filling nitrogen to a pressure of 40 bar, after which the internal gas of the reactor was completely removed under vacuum, and then the reactor was adjusted to about 1.2 bar by carbon monoxide. A reactant composition comprising 32.5 wt% of methanol, 56.4 wt% of acetic acid, 6.5 wt% of methyl iodide and 4.6 wt% of water was prepared, and 45 g of the reactant composition was taken by a syringe. The ball valve plug in the upper portion of the reactor was opened, and the valve was opened slowly such that carbon monoxide was vented out slowly. Then, the valve was completely opened, and the reactant in the syringe was rapidly introduced into the reactor. Next, the ball valve was closed and completely sealed with the plug, and stirring at 1250 rpm was started. The internal temperature of the reactor was elevated to 190°C, and when the temperature was stabilized, the valve connected to the reactor was opened and carbon monoxide adjusted to 40 bar was introduced into the reactor to initiate the reaction. After the reaction, a carbon monoxide gas line was blocked, and the reactor was separated from a heating mantle and cooled rapidly with cold water, and the weight of the product was measured. The reactor was dissembled, and the product was analyzed by GC. Furthermore, the 6 mm PTFE Raschig ring rhodium complex ion catalyst was recovered, washed with methanol, dried completely in a vacuum dryer at 80°C, and reused as a catalyst in the next reaction. The first reaction performed as described above was defined as a zero-order reaction, and the 6 mm PTFE Raschig ring rhodium complex ion catalyst obtained therein was used as a catalyst in the next first-order reaction. In the next reaction, among the reaction preparation procedures as described above, the rhodium salt was not introduced, and the 6 mm PTFE Raschig ring rhodium complex ion catalyst obtained in the zero-order reaction was introduced, and a predetermined amount of the reactant composition having the same composition as described above was introduced according to a predetermined order, after which the reaction was performed and the weight of the product was measured and analyzed. The results were defined as first-order reaction results, and the catalyst used was recovered, washed with methanol, dried, and then used in a second-order reaction. Table 22 below shows the results of measuring the conversion rate of methanol and the yields of acetic acid, methyl acetate and water in the products obtained by the acetic acid synthesis reaction performed using the 6 mm PTFE Raschig ring rhodium complex ion catalyst. These results are merely used to compare relative results according to the DOG % of the same catalyst support, because it is obvious that the liquid stirred by the magnetic bar comes into contact with carbon monoxide via the Raschig ring placed on the mesh, and thus mass transfer is not smooth and the reaction rate is very slower than the reaction rate of a homogeneous catalyst. When DOG% was 0.9%, the conversion rate of methanol in the first-order reaction was 90% or less, and the yield of acetic acid decreased and the yield of methyl acetate was 42% or more, indicating that the rhodium complex ion did not bond in the zero-order reaction, because the ligand was less grafted due to low DOG%. In this case, the rhodium complex ions were mostly lost as a solution, and thus the acetic acid synthesis reaction did not occur in the first-order reaction. In addition, ICP analysis indicated that there was no loss of the rhodium ion. When DOG% was 2% or more, the conversion rate of methanol was 99% or more, the yield of acetic acid was 75% or more, and the loss of rhodium was very low. However, when the dose of gamma rays was 105 kGy, the yield of acetic acid slightly decreased, the yield of methyl acetate increased, and thus the yield of water also increased, thus reducing the activity of the catalyst. It is believed that this phenomenon occurred because the ligand grafted on the surface of PTFE was significantly detached in the zero-order reaction due to weak bonding caused by the corrosion of PTFE caused by excessive gamma-ray irradiation. The results are summarized in Table 22 below.

**Table 22**

| Example 10 | | | | | | | | | | |
|---|---|---|---|---|---|---|---|---|---|---|
| Experi -nal order | Catalyst | Rh | Reaction conditions | Reactnt | Product | Convesion % | Yield % | | | Rh Loss % |
| | No/g/EA/DOG%/RhCl₃ (mg) | ppm | °C/bar/hr | g | g | MeOH | AcOH | AcCMe | H₂O | |
| 1 | PTFE6-4VP/AcAc35-IM0.25-50-5KG/15(115/20.165/100/0.13/) | 400 | 190/40/3 | 45.28 | 45.26 | 89.59 | -16.89 | 42.20 | 59.42 | - |
| 1 | PTFE6-4VP/AcAc10-IM0.50-50-5KG/15(119/20.188/100/2.51/) | 400 | 190/40/3 | 45.01 | 55.70 | 99.48 | 75.18 | 9.52 | 9.93 | 1.55 |
| 1 | PTFE6-4VP/AcAc10-IM0.10-50-6KG/15(144/20.161/100/3.66/) | 400 | 190/40/3 | 45.19 | 55.69 | 99.66 | 77.60 | 9.38 | 9.62 | 1.09 |
| 1 | PTFE6-4VP/AcAc10-IM0.10-50-7KG/15(171/19.935/100/4.52/ | 400 | 190/40/3 | 45.83 | 52.51 | 99.16 | 41.63 | 22.29 | 28.07 | 5.98 |

As described above, according to the present invention, a fixed-bed bubble column reactor can be easily designed. In addition, a special device for catalyst separation is not required, and thus the device manufacturing cost can be saved, the operating cost can be reduced due to process simplification, and productivity can be greatly increased.

Although the specific embodiments of the present invention have been disclosed for illustrative purposes, those skilled in the art will appreciate that various modifications, additions and substitutions are possible without departing from the scope and spirit of the invention as disclosed in the accompanying claims.

## Claims

1. A heterogeneous catalyst for producing acetic acid by carbonylation of methanol, wherein a rhodium complex ion is ionically bonded to an insoluble catalyst support, and the insoluble catalyst support includes a fluoropolymer having a quaternary pyridine radical alone or in combination with an acetate radical grafted on a surface thereof.

2. The heterogeneous catalyst of claim 1, wherein grafting the pyridine radical as a ligand on the surface of the fluoropolymer is achieved by diluting vinylpyridine in a solvent to a concentration of 20 wt% to 70 wt%, adding Mohr's salt as a polymerization inhibitor thereto, and irradiating cobalt gamma rays.

3. The heterogeneous catalyst of claim 1, wherein grafting the pyridine radical and the acetate group as a ligand to the surface of the fluoropolymer is achieved by mixing more than 0 mol% but not more than 35 mol% of vinyl acetate with vinylpyridine to obtain a vinylpyridine/vinyl acetate mixture, diluting the vinylpyridine/vinyl acetate mixture in a solvent to a concentration of 20 wt% to 70 wt%, adding Mohr's salt as a polymerization inhibitor thereto, and irradiating cobalt gamma rays.

4. The heterogeneous catalyst of any one of claims 1, wherein the fluoropolymer for grafting is any one selected from the group consisting of PTFE (polytetrafluoroethylene), PFA (perfluoroalkoxy resin), and a mixture thereof.

5. The heterogeneous catalyst of claim 4, wherein the polymer is shaped into any one or more selected from the group consisting of a sphere shape, a Raschig ring shape, a Pall ring shape, a tri-pack shape, a tellerette shape, and a saddle ring shape.

6. The heterogeneous catalyst of claim 2, wherein the Mohr's salt is added in an amount of 0.1 wt% to 5 wt%.

7. The heterogeneous catalyst of claim 3, wherein the Mohr's salt is added in an amount of 0.1 wt% to 5 wt%.

8. The heterogeneous catalyst of claim 2, wherein divinylbenzene as a crosslinking agent is used in an amount of 0.1 mol% to 5 mol% based on the ligand compound in grafting of the ligand on the surface of the fluoropolymer in order to increase the ligand grafted on the surface of the fluoropolymer.

9. The heterogeneous catalyst of claim 3, wherein divinylbenzene as a crosslinking agent is used in an amount of 0.1 mol% to 5 mol% based on the ligand compound in grafting of the ligand on the surface of the fluoropolymer in order to increase the ligand grafted on the surface of the fluoropolymer.

10. The heterogeneous catalyst of claim 2, wherein cobalt gamma rays are irradiated with a total dose of 40 kGy to 150 kGy such that the ligand compound is radically grafted onto the surface of the fluoropolymer.

11. The heterogeneous catalyst of claim 3, wherein cobalt gamma rays are irradiated with a total dose of 40 kGy to 150 kGy such that the ligand compound is radically grafted onto the surface of the fluoropolymer.

12. The heterogeneous catalyst of claim 4, wherein one obtained by shaping a composition including the fluoropolymer and any one or more selected from the group consisting of alumina, clay, carbon, and silica is preferably used as the support.

13. The heterogeneous catalyst of claim 4, wherein one obtained by applying the fluoropolymer on a shaped surface of any one selected from the group consisting of alumina, clay, carbon, and silica is preferably used as the support.
